# EUROPEAN PATENT APPLICATION

(11) **EP 4 029 876 A1**
(43) Date of publication of application: **20.07.2022**
(21) Application number: 20864099.5
(22) Date of filing: 08.09.2020
(51) Int. Cl.: C07K 14/435, C12N 15/31, C12N 15/10

(54) **METHOD FOR IDENTIFYING SUBSTANCE CAPABLE OF AFFECTING AGING**

(30) Priority: 09.09.2019 CN 201910846309
(71) Applicant: Zhejiang Baishoukangdi Co. Ltd., Hangzhou, Zhejiang 310053 (CN)
(72) Inventor: MIN, Junxia, Hangzhou, Zhejiang 310053 (CN)
(74) Representative: Böhm, Brigitte
(86) International application number: PCT/CN2020/114024
(87) International publication number: WO 2021/047507

(57) **Abstract**

Provided is a method for identifying a substance capable of affecting aging, the method comprising: determining the effect of a candidate substance on the activity and/or expression of Zip11; and identifying the candidate substance that changes the activity and/or expression of Zip 11 as a substance capable of affecting aging. Further provided is the use of a substance capable of increasing the activity and/or expression quantity of Zip 11 for preparing a medicine, wherein the medicine is used for delaying or ameliorating aging.

## Description

### TECHNICAL FIELD

The present application relates to the field of biomedicine, and in particular, relates to a method for identifying a substance capable of affecting aging, where the substance can affect the activity and/or expression of Zip 11.

### BACKGROUND ART

Aging, as an inevitable biological process, is usually characterized by the decline of physiological functions, which then leads to morbidity and death. During this gradual process, the decline of physiological functions is manifested as an overall decrease in physical fitness and brain power. The aging of functions, tissues, and cells is prevalent in all multicellular organisms. However, the rate of aging varies greatly among individuals, and environmental factors and genetic factors together contribute to changes in the rate of aging. The analytical investigations on the whole human genome have found that the genetic variation of certain genes such as ApoE, Sirt1, Foxo3 or Nrf3 is significantly related to the longevity or health of the individuals, however, there are problems with regional limitation, safety or effectiveness, which greatly limits the application of related products. Hence, it is necessary to develop new longevity-related genes with an excellent potential. (See Zeng Y, etc. Sex Differences in Genetic Associations with Longevity, JAMA Netw Open. 2018 Aug; 1(4).pii: el81670.) .

### SUMMARY OF THE INVENTION

The present application has unexpectedly discovered that *Zip11* gene, which encodes a member of the zinc transporter solute carrier family 39 (SLC39), plays a crucial role in many aspects, such as inflammation, tumors, and metabolism, and it is also highly expressed in many organs, such as the small intestine, the stomach, the reproductive system and the liver. By regulating the expression of *Zip11* proteins, related reactions can be regulated to affect the aging level and lifespan of an organism. Therefore, the Zip11 gene is a longevity-related gene with an excellent potential.

The present application provides a method for identifying a substance capable of affecting aging. The method comprises: determining an effect of a candidate substance on an activity and/or expression of Zip11; and identifying the candidate substance, that changes the activity and/or expression of the Zip11, as the substance capable of affecting the aging. The present invention further provides use of a substance capable of increasing an activity and/or expression quantity of *Zip11* for the preparation of a drug for delaying or ameliorating aging.

In one aspect, the present application provides a method for identifying a substance capable of affecting aging. The method comprises: determining an effect of a candidate substance on an activity and/or expression of Zip11; and identifying the candidate substance, that changes the activity and/or expression of the Zip11, as the substance capable of affecting the aging.

In some embodiments, determining the effect of the candidate substance on the activity and/or expression of the Zip11 comprises: determining an effect of the candidate substance on a nucleic acid expression level and/or activity of the Zip11.

In some embodiments, the nucleic acid expression level and/or activity of the Zip11 is determined by using a substance selected from the group consisting of: a primer capable of specifically amplifying the Zip11, and a nucleic acid probe capable of specifically recognizing the Zip11.

In some embodiments, determining the effect of the candidate substance on the activity and/or expression of the Zip11 comprises: determining the effect of the candidate substance on a protein expression level and/or activity of the Zip11.

In some embodiments, the protein expression level and/or activity of the Zip11 is determined by using a substance selected from the group consisting of: a reagent specifically recognizing a protein of the Zip11, and a substance capable of determining a protein activity of the Zip11.

In some embodiments, a candidate substance that increases the activity and/or expression of the Zip11 is identified as a substance capable of delaying or ameliorating the aging.

In some embodiments, a candidate substance that reduces the activity and/or expression of the Zip11 is identified as a substance capable of accelerating or deteriorating the aging.

In some embodiments, the method comprises administering the candidate substance to a non-human animal, an organ, a tissue and/or a cell, determining the effect of the candidate substance on the activity and/or expression of the Zip11 in the non-human animal, the organ, the tissue and/or the cell, and identifying the candidate substance, that changes the activity and/or expression of the Zip11, as the substance capable of affecting the aging.

In some embodiments, the method is an *in vitro* or *ex vivo* method.

In some embodiments, a condition of the aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

In some embodiments, the body physique condition is evaluated by detecting a spine form. In some embodiments, the hair condition is evaluated by detecting hair loss. In some embodiments, the immune level condition is evaluated by detecting a cytokine level. In some embodiments, the tumor-bearing condition is evaluated by detecting a tumor volume, tumor progression, and/or an occurrence of tumor cells. In some embodiments, the liver condition is evaluated by detecting a alanine aminotransferase activity, an aspartate aminotransferase activity and /or a liver/body weight ratio. In some embodiments, the spleen condition is evaluated by detecting a spleen/body weight ratio. In some embodiments, the reproductive capacity condition is evaluated by detecting a proportion of fertile eggs. In some embodiments, the exercise capacity condition is evaluated from fatigue rotarod test results. In some embodiments, the lifespan condition is evaluated by detecting a survival time. In some embodiments, the cognitive capacity condition is evaluated by an open field test.

In another aspect, the application provides use of a substance capable of increasing an activity and/or expression quantity of Zip11 for the preparation of a drug for delaying or ameliorating aging.

In some embodiments, delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity and/or increasing a behavioral cognitive capacity.

In some embodiments, the increasing refers to an increase of at least 15 times in the activity and/or expression quantity of the Zip11 after administration of the substance as compared to that of without administration of the substance.

In some embodiments, the activity and/or expression quantity of the Zip11 comprises an activity and/or expression quantity at a nucleic acid level of the Zip11 and/or at a protein level of the Zip11.

In another aspect, the present application provides a system for identifying a substance capable of affecting aging, comprising a substance capable of determining an effect of a candidate substance on an activity and/or expression of Zip11.

In some embodiments, the system comprises a substance capable of determining a nucleic acid expression level and/or activity of the Zip11.

In some embodiments, the substance capable of determining the nucleic acid expression level and/or activity of the Zip11 comprises: a primer capable of specifically amplifying the Zip11 and/or a probe capable of specifically recognizing the Zip11.

In some embodiments, the system comprises a substance capable of determining a protein expression level and/or activity of the Zip11.

In some embodiments, the substance capable of determining the protein expression level and/or activity of the Zip11 comprises a reagent capable of specifically recognizing a protein of the Zip11 and/or a reagent capable of determining a protein activity of the Zip11.

In another aspect, the present application provides use of a nucleic acid molecule encoding Zip11, or an expression product thereof, for the preparation of a drug for delaying or ameliorating aging.

In some embodiments, delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity and/or increasing a behavioral cognitive capacity.

In some embodiments, the nucleic acid molecule comprises a nucleotide sequence as set forth in any one in the group consisting of SEQ ID NO.: 1.

In another aspect, the present application further provides a method for delaying or ameliorating aging, comprising administering an effective amount of a nucleic acid molecule encoding Zip11, or an expression product thereof, to a subject in need thereof.

In some embodiments, delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, delaying a time point of exercise capacity degeneration, and/or increasing a behavioral cognitive capacity.

In some embodiments, the nucleic acid molecule comprises a nucleotide sequence as set forth in SEQ ID NO: 1.

In another aspect, the present application further provides a method for identifying an aging condition in a subject, comprising: determining an activity and/or expression quantity of Zip11 in the subject.

In some embodiments, the activity and/or expression quantity of the Zip11 comprises a nucleic acid expression level and/or activity of the Zip11, and/or a protein expression level and/or activity of the Zip11.

In some embodiments, the nucleic acid expression level and/or activity of the Zip11 in the subject is determined by using a substance selected from the group consisting of: a primer capable of specifically amplifying the Zip11, and a nucleic acid probe capable of specifically recognizing the Zip11.

In some embodiments, the protein expression level and/or activity of the Zip11 in the subject is determined by using a substance selected from the group consisting of: a reagent specifically recognizing a protein of the Zip11, and a substance capable of determining a protein activity of the Zip11.

In some embodiments, a condition of the aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

In some embodiments, the body physique condition is evaluated by detecting a spine form. In some embodiments, the hair condition is evaluated by detecting hair loss. In some embodiments, the immune level condition is evaluated by detecting a cytokine level. In some embodiments, the tumor-bearing condition is evaluated by detecting a tumor volume, tumor progression, and/or an occurrence of tumor cells. In some embodiments, the liver condition is evaluated by detecting a alanine aminotransferase activity, an aspartate aminotransferase activity and /or a liver/body weight ratio. In some embodiments, the spleen condition is evaluated by detecting a spleen/body weight ratio. In some embodiments, the reproductive capacity condition is evaluated by detecting a proportion of fertile eggs. In some embodiments, the exercise capacity condition is evaluated from fatigue rotarod test results. In some embodiments, the lifespan condition is evaluated by detecting a survival time. In some embodiments, the cognitive capacity condition is evaluated by an open field test.

In another aspect, the present application further provides use of a substance determining an activity and/or expression quantity of Zip11 for the preparation of a reagent for identifying an aging condition in a subject.

In some embodiments, the activity and/or expression quantity of the Zip11 comprises a nucleic acid expression level and/or activity of the Zip11, and/or a protein expression level and/or activity of the Zip11.

In some embodiments, the substance comprises: a primer capable of amplifying the Zip11, a nucleic acid probe capable of specifically recognizing the Zip11, a reagent capable of specifically recognizing a protein of the Zip11, and/or a substance capable of determining a protein activity of the Zip11.

In some embodiments, a condition of the aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

In some embodiments, the body physique condition is evaluated by detecting a spine form. In some embodiments, the hair condition is evaluated by detecting hair loss. In some embodiments, the immune level condition is evaluated by detecting a cytokine level. In some embodiments, the tumor-bearing condition is evaluated by detecting a tumor volume, tumor progression, and/or an occurrence of tumor cells. In some embodiments, the liver condition is evaluated by detecting an alanine aminotransferase activity, an aspartate aminotransferase activity and /or a liver/body weight ratio. In some embodiments, the spleen condition is evaluated by detecting a spleen/body weight ratio. In some embodiments, the reproductive capacity condition is evaluated by detecting a proportion of fertile eggs. In some embodiments, the exercise capacity condition is evaluated from fatigue rotarod test results. In some embodiments, the lifespan condition is evaluated by detecting a survival time. In some embodiments, the cognitive capacity condition is evaluated by an open field test.

In another aspect, the present application provides a system for identifying an aging condition in a subject, comprising a substance capable of determining an expression and/or activity of Zip11.

In some embodiments, the system comprises a substance capable of determining a nucleic acid expression level and/or activity of the Zip11.

In some embodiments, the substance capable of determining the nucleic acid expression level and/or activity of the Zip11 comprises: a primer capable of amplifying the Zip11 and/or a nucleic acid probe capable of specifically recognizing the Zip11.

In some embodiments, the system comprises a substance capable of determining a protein expression level and/or activity of the Zip11.

In some embodiments, the substance capable of determining the protein expression level and/or activity of the Zip11 comprises: a substance capable of specifically recognizing a protein of the Zip11 and/or a substance capable of determining a protein activity of the Zip11.

In some embodiments, a condition of the aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

In some embodiments, the body physique condition is evaluated by detecting a spine form. In some embodiments, the hair condition is evaluated by detecting hair loss. In some embodiments, the immune level condition is evaluated by detecting a cytokine level. In some embodiments, the tumor-bearing condition is evaluated by detecting a tumor volume. In some embodiments, the liver condition is evaluated by detecting a alanine aminotransferase activity, an aspartate aminotransferase activity and /or a liver/body weight ratio. In some embodiments, the spleen condition is evaluated by detecting a spleen/body weight ratio. In some embodiments, the reproductive capacity condition is evaluated by detecting a proportion of fertile eggs. In some embodiments, the exercise capacity condition is evaluated from fatigue rotarod test results. In some embodiments, the lifespan condition is evaluated by detecting a survival time. In some embodiments, the cognitive capacity condition is evaluated by a detection open field test.

In another aspect, the present application provides a method for assessing an effect of a candidate substance on an aging condition in a subject, comprising:
1) administering the candidate substance to be assessed to the subject;
2) comparing changes in an expression and/or activity of Zip11 in the subject before and after the administering; and
3) assessing the effect of the candidate substance on the aging condition in the subject based on the changes in the expression and/or activity of the Zip11.

In some embodiments, the activity and/or expression quantity of the Zip11 comprises an activity and/or expression quantity at a nucleic acid level of the Zip11 and/or at a protein level of the Zip11.

In some embodiments, the candidate substance delays or ameliorates the aging condition in the subject in the case of up-regulated expression and/or increased activity of the Zip11 in the subject after the administrating.

In some embodiments, delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, delaying a time point of exercise capacity degeneration, and/or increasing a behavioral cognitive capacity.

In some embodiments, the candidate substance accelerates or deteriorates the aging condition in the subject in the case of down-regulated expression and/or reduced activity of the Zip11 in the subject after the administrating.

In some embodiments, accelerating or deteriorating the aging comprises: exacerbating spinal curvature, exacerbating hair loss, decreasing a cytokine level, weakening immunity, deteriorating or recurring tumors, shortening a lifespan, exacerbating a liver injury, exacerbating a spleen injury, exacerbating decreased fertility, reducing an exercise capacity, and/or reducing a behavioral cognitive capacity.

In another aspect, the present application further provides a non-human animal or a part thereof, in which Zip11 is over-expressed.

In some embodiments, the non-human animal or the part thereof comprises an additional number of copies of a Zip11 gene as compared to a corresponding wild type.

In some embodiments, an expression of the Zip11 gene is regulated by a tissue-specific promoter.

In some embodiments, the additional number of copies of the Zip11 gene are specifically expressed in the liver.

In some embodiments, the tissue-specific promoter comprises a nucleotide sequence as set forth in SEQ ID No. 1.

In some embodiments, the non-human animal or the part thereof shows a phenotype delaying or ameliorating an aging condition.

In some embodiments, delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, delaying a time point of exercise capacity degeneration, and/or increasing a behavioral cognitive capacity.

In another aspect, the present application further provides a non-human animal or a part thereof, in which an expression and/or function of a Zip11 gene is disrupted.

In some embodiments, the Zip11 gene is at least partially knocked out.

In some embodiments, an exon 5 of the Zip11 gene is at least partially knocked out.

In some embodiments, the non-human animal or the part thereof shows a phenotype accelerating or deteriorating an aging condition.

In some embodiments, accelerating or deteriorating the aging comprises: exacerbating spinal curvature, exacerbating hair loss, decreasing a cytokine level, weakening immunity, deteriorating or recurring tumors, shortening a lifespan, exacerbating a liver injury, exacerbating a spleen injury, exacerbating decreased fertility, reducing an exercise capacity, and/or reducing a behavioral cognitive capacity.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the present invention involved in the present application are listed in the appended claims. The characteristics and advantages of the present invention involved in the present application can be better understood by referring to the exemplary embodiments and the accompanying drawings described in detail below. A brief description of the drawings is as follows:
FIG. 1A shows the sequence alignment results of human Zip11 and zebrafish Zip11.
FIG. 1B shows a process of constructing Zip11 gene knockout zebrafish described in the present application.
FIG. 2 shows the morphological characteristics of *Zip11*^{*-*/*-*} zebrafish described in the present application.
FIG. 3 shows the survival curves of *Zip11*^{*-*/*-*} zebrafish described in the present application.
FIG. 4 shows reproduction rates of F2-generation *Zip11*^{*-*/*-*} zebrafish described in the present application.
FIG. 5 shows a process of constructing a *Zip11*^{*-*/*-*} mouse model described in the present application, with A showing a schematic diagram of gene knockout, B showing a vector structure, and C showing Zip11 mRNA levels.
FIGs. 6 and 7 show hair loss in *Zip11*^{*-*/*-*} mice described in the present application.
FIG. 8 shows hair loss and spleen conditions in *Zip11*^{*-*/*-*} mice described in the present application.
FIG. 9 shows spleen slice conditions in *Zip11*^{*-*/*-*} mouse described in the present application.
FIG. 10 shows liver slice conditions in *Zip11*^{*-*/*-*} mice described in the present application.
FIG. 11 shows the appearance and genotype electropherogram of a *Zip11*^{*-*/*-*} mouse described in the present application.
FIG. 12 shows tumor growth conditions in *Zip11*^{*-*/*-*} mice described in the present application.
FIG. 13 shows secretion levels of inflammatory factors in *Zip11*^{*-*/*-*} mice described in the present application.
FIG. 14 shows exercise capacities in *Zip11*^{*-*/*-*} mice described in the present application.
FIG. 15 shows a process of constructing a *ZIP11 KI* mouse model described in the present application.
FIG. 16 shows mRNA expression levels of *ZIP11* in wild-type mice with inflammation induced by Concanavalin A.
FIG. 17 shows a liver comparison (A), a liver/body weight ratio (B), as well as ALT (C) and AST (D) levels in wild-type mice and *ZIP11 KI* mice.
FIG. 18 shows liver cell morphologies in *ZIP11 KI* mice under different staining methods.
FIG. 19 shows that vitamins can increase the expression levels of *Zip11* gene in human-derived cells.
FIG. 20 shows weight gains in *Zip11*^{*-*/*-*} mice fed with a high-fat diet.
FIG. 21 shows a decrease of learning and cognitive capacities in *Zip11*^{*-*/*-*} male mice.
FIG. 22 shows a movement trajectory diagram of mice in an open field test.
FIG. 23 shows an increase of p53 protein levels in *Zip11*^{*-*/*-*} mice.
FIG. 24 shows a decrease in M1 macrophages (A) and an increase in M2 macrophages (B), in *Zip11*^{*-*/*-*} mice stimulated by LPS.
FIG. 25 shows a decrease of bilirubin in *ZIP11*-overexpressed mice.
FIG. 26 shows an increase of bilirubin in *Zip11*^{*-*/*-*} mice.

### DETAILED DESCRIPTION OF THE INVENTION

The embodiments of the invention of the present application will be illustrated by specific examples below. Those familiar with this technology can easily understand other advantages and effects of the invention of the present application from the content disclosed in the specification.

### Zip11 nucleic acid, Zip11 protein

In the present application, the terms *"Zip11",* "ZIP11", "ZIP-11", and "SLC39A11" are used interchangeably and generally refer to one of the members of a zinc transporter solute carrier family (SLC39). These terms may also refer to genes encoding the Zip11 protein. The *Zip11* described in the present application may comprise human *ZIP11,* zebrafish *Zip11,* and/or mouse Zip11, or functional fragments or variants thereof, and may also comprise homologues in other species. For example, the human ZIP11 may contain a fragment of 342 amino acids, with an amino acid sequence that can be found in NCBI under Accession No. NP_001153242.1, and it is encoded by a gene as set forth in Gene ID: 201266 of the NCBI database; a zebrafish *Zip11* protein may contain 123 amino acids, with an amino acid sequence that can be found in NCBI under Accession No.: XP_021335877.1, and a zebrafish *Zip11* gene sequence can be found in NCBI under Accession No. NC_007123.7; the mouse Zip11 may contain 342 amino acids, with an amino acid sequence that can be find in NCBI under Accession No. NP_001159975.1; and a mouse *Zip11* gene can be found in the MGI database under ID: 1917056. In the present application, Zip11 gene knockout may be represented as *Zip11* KO *or Zip11* KO or *Zip11*^{*-*/}*⁻.* Zip11 gene knock in or overexpression may be represented as *ZIP11 KI* or *Zip11 KI.*

By means of genome-wide association analysis, the inventor of the present application compared and analyzed the whole genomes of 2178 centenarians (564 males, 1614 females) and 2299 (773 males, 1526 females) middle-aged people in a control group. A number of sequence variations and single nucleotide polymorphisms (SNPs) were found to be significantly related to longevity (p<10⁵) in the females and males, respectively. Various SNP genes comprising *ZIP11* were found to be significantly associated with a male aging condition (See Zeng Y, etc. Sex Differences in Genetic Associations with Longevity, JAMA Netw Open. 2018 Aug; 1 (4). Pii: el81670.).

### Aging condition

In the present application, the term "aging" generally refers to the accumulation of changes in an organism over time. Such changes may range from changes that affect genetics and cell functions to changes that affect the functions of organs, organ systems, or entire organisms. Aging particularly refers to changes that occur after an organism has reached biological maturity and may develop until the organism eventually dies. The aging condition may be determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

The inventor of the present application has found that the aging is delayed or ameliorated in individuals with increased activity and/or expression of Zip11. "Delaying or ameliorating" generally refers to preventing the progression of or treating a condition, a disease, a disorder, or a phenotype (comprising abnormalities or symptoms), in order to reduce, decrease or eliminate them. Delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity and/or delaying a time point of exercise capacity degeneration, and/or increasing a behavioral cognitive capacity. Preventing or treating spinal curvature may comprise reducing the degree of spinal curvature or straightening the spinal column. Preventing or treating hair loss may comprise reducing the degree of hair loss or preventing the hair loss. The cytokine may comprise IL-6, IL-2, IL-1-β, IL-7, IL-10, IL-12, TNF-α, IFN-α, IFN-β, IFN-γ, GM- CSF, and G-CSF. Enhancing immunity may comprise increasing the cytokine level. Enhancing immunity may comprise increasing the percentage of M1 type macrophages and reducing the percentage of M2 type macrophages. Preventing or treating tumors may comprise reducing tumor volume or preventing the occurrence of tumors. Prolonging the lifespan may comprise reducing the activity/expression level of aging-related genes. Prolonging the lifespan may comprise prolonging a survival time or increasing a population survival rate. Preventing or treating the liver injury may comprise increasing the sensitivity to liver inflammation. For example, the sensitivity to liver inflammation may be evaluated based on the levels of alanine aminotransferase and aspartate aminotransferase, and the morphology of liver cells. Preventing or treating the decreased fertility may comprise increasing the proportion of fertile eggs. Increasing the exercise capacity may comprise prolonging the time for maintaining exercises, delaying the time point of exercise capacity degradation and the like. Increasing the cognitive capacity may comprise boosting the desire to explore an unknown space (for example, in an open field test).

The inventor of the present application has also found that aging is accelerated or deteriorated in individuals with reduced activity and/or expression of Zip11. "Accelerating or deteriorating" generally refers to increasing, improving, or maintaining a condition, a disease, a disorder, or a phenotype (comprising abnormalities or symptoms). Accelerating or deteriorating the aging comprises: accelerating or deteriorating spinal curvature, accelerating or deteriorating hair loss, decreasing a cytokine level, weakening immunity, accelerating or deteriorating tumors, shortening a lifespan, accelerating or deteriorating a liver injury, accelerating or deteriorating a spleen injury, accelerating or deteriorating decreased fertility, reducing an exercise capacity, and/or reducing a behavioral cognitive capacity. Accelerating or deteriorating the spinal curvature may comprise increasing the degree of spinal curvature. Accelerating or deteriorating the hair loss may comprise abnormal to complete hair loss on the head or body. The cytokine may comprise IL-6, IL-2, IL-1-β, IL-7, IL-10, IL-12, TNF-α, IFN-α, IFN-β, IFN-r, GM-CSF, and G-CSF. Weakening the immunity may comprise reducing the cytokine level. Weakening the immunity may comprise reducing the percentage of M1 type macrophages and increasing the percentage of M2 type macrophages. Accelerating or deteriorating the tumor may comprise increasing a tumor volume. Shortening the lifespan may comprise shortening a survival time or reducing a quiet population survival rate. Accelerating or deteriorating the liver injury may comprise liver enlargement. Accelerating or deteriorating the liver injury may comprise spleen enlargement. Accelerating or deteriorating the decline in fertility may comprise reducing the proportion of fertile eggs. Reducing the exercise capacity may comprise shortening the time for maintaining exercises or advancing the time point of exercise capacity degradation. Reducing cognitive capacity may comprise reducing the desire to explore an unknown space (for example, in an open field test).

The changes in the activity and/or expression of the Zip11 may be determined using methods known in the art, comprising immunohistochemical methods, PCR, RT-PCR, in situ hybridization, Southern blot, Western blot, Northern blot, spectrophotometry, gene chip, flow cytometry (FACs), protein chip, DNA sequencing and ELISA, etc. In some cases, the method may comprise a primer capable of specifically amplifying the Zip11, and a nucleic acid probe capable of specifically recognizing the Zip11. The probe may specifically bind to a specific site on the *Zip11* gene, but does not specifically bind to other genes other than the *Zip11* gene, so as to "specifically recognize" the *Zip11* gene. Moreover, the probe has a detectable signal. The primer may amplify the *Zip11* gene without amplifying other genes, that is, "specific amplification". In some cases, the candidate substance consists of oligonucleotide fragments. Such fragments should be long enough to be possibly hybridized with a sample RNA or DNA. Such fragments may be 10-40 nucleotides long or longer, such as 45, 50, 100, 500, or even full length. In some cases, the method may comprise a reagent specifically recognizing a protein of the Zip11, and a substance capable of determining a protein activity of the Zip11. The substance may be a protein binding molecule, such as an antibody or a ligand, which may specifically bind to Zip11 or a protein fragment thereof.

### Uses, methods and systems

The present application provides a method for identifying a substance capable of affecting aging. The method may comprise: determining an effect of a candidate substance on an activity and/or expression; and identifying the candidate substance, that changes the activity and/or expression, as the substance capable of affecting the aging. In some cases, the method is applicable to drug screening. For example, the substance increasing the activity and/or expression of the *Zip11* may be identified as the substance capable of affecting the aging, for use in the preparation of a drug for delaying or ameliorating the aging. For another example, the substance reducing the activity and/or expression of the *Zip11* may be identified as the substance capable of affecting the activity and/or expression quantity of *Zip11.* This substance may have toxic or side effects.

The present application provides a system for identifying a substance capable of affecting aging. The system may comprise a substance capable of determining an effect of a candidate substance on an activity and/or expression of *Zip11.* The system may comprise a kit. The kit contains the substance which is appropriately packaged and is capable of affecting the aging as described in the present application, and may comprise written materials such as instructions for use, clinical study discussions, and side effects lists. Such a kit may also comprise information, such as scientific literature references, package insert materials, clinical trial results, and/or summaries of these similar information, which indicate or confirm the activity and/or advantages of the composition, and/or describe a dosage regimen, administration, side effects, drug interactions, or other information useful to healthcare providers. The kit may further contain another agent. In some cases, the substance capable of affecting the aging and the agent are provided as separate compositions in separate containers in the kit. In some cases, the substance capable of affecting the aging and the agent are provided as a single composition in a container of the kit. Appropriate packaging and additional supplies (such as measuring cups for liquid preparations, foil packaging to minimize exposure to air, etc.) are known in the art and may be comprised in the kit. In some cases, the system may be provided, sold, and/or promoted to related personnel, comprising healthcare supplies, physicians, nurses, pharmacists, prescribers, drug developers, drug manufacturers, and the like. In other cases, the system may also be sold directly to consumers.

The present application provides use of a nucleic acid molecule encoding *Zip11,* or an expression product thereof, for the preparation of a drug for delaying or ameliorating aging. In some cases, the nucleic acid molecules encoding the *Zip11,* or an expression product thereof, may be used in the preparation of a drug for gene therapy. The drug may comprise a vector capable of expressing the nucleic acid molecule. The vector may be a nucleic acid molecule capable of proliferating another nucleic acid to which the is linked. The term comprises a vector of a self-replicating nucleic acid structure, and a vector that is incorporated into a genome of a host cell into which the former vector has been introduced. Certain vectors are capable of directing the expression of nucleic acids to which these vectors are operably linked. The drug may comprise a substance that has the effect of enhancing an expression, for example acting on the promoter or enhanced region of the *Zip11,* or a substance that has the effect of enhancing the transcription of the *Zip11* gene to mRNA, or a substance exhibiting the same effect by means of transcription factors and the like in cells. The drugs may also be placed in a container together with a packaging material, and the latter provides instructions on the usage of these drugs. In general, these instructions will comprise an exact statement describing the concentration of a agent, and in some embodiments may comprise the relative amounts of excipient ingredients or diluents (for example, water, saline, or PBS) necessary to reconstruct a pharmaceutical composition.

The present application provides a method for delaying or ameliorating aging, comprising administering an effective amount of a nucleic acid molecule encoding *Zip11,* or an expression product thereof, to a subject in need thereof. In the present application, the term " subj ect" generally refers to any human or non-human animal. The non-human animal comprises all vertebrates, such as mammals and non-mammals, for example, non-human primates, sheep, dogs, cats, cows, horses, chickens, fish, mice, rabbits, amphibians, and reptiles.

In the present application, the term "effective amount" generally refers to an amount of an agent that is non-toxic but sufficient to provide a desired biological result. This amount is sufficient to reduce and/or ameliorate the severity and/or duration or symptoms of a disorder (aging symptoms or related disorders), prevent the deterioration of the disorder, decline the disorder, and prevent the recurrence, development or onset of one or more symptoms related to the disorder, or enhance or improve the preventive or therapeutic effect of another therapy (for example, a preventive or therapeutic agent). For example, the effective amount may be an amount capable of delaying or ameliorating the aging.

In the present application, the term "administration" generally refers to a method of administering a certain dose of substance or pharmaceutical composition to a subject (for example, a patient with symptoms of aging). The administration may be carried out by any suitable means, comprising but not limited to injection (subcutaneous, intravenous, parenteral, intraperitoneal, intrathecal and other injections), oral administration, inhalation, and rectal and intradermal administration.

For example, the substance capable of increasing the activity and/or expression quantity of the *Zip11,* the nucleic acid molecule encoding the Zip11 or an expression product thereof, and the vector described in the present application may be separately or jointly prepared into a form suitable for oral administration. For example, tablets or capsules prepared by traditional methods and added with pharmaceutically acceptable excipients which are for example binding agents, fillers, lubricants, disintegrants or wetting agents. A method for packaging the tablets is known to those skilled in the art. Liquids for oral administration may be prepared in the form of, for example, solutions, syrups or suspensions, or they may be provided in the form of dry products, which are combined with water or other suitable excipients and carriers before use. Various delivery systems are known and may be used to administer the compounds of the present invention, which, for example, may be delivered by being encapsulated within liposomes, microparticles and microcapsules, via recombinant cells capable of expressing the compounds, via receptor-mediated endocytosis, by constructing nucleic acids as part of retroviruses or other vectors, etc.

In some cases, a nucleic acid molecule encoding *Zip11* or an expression product thereof may be administered by a gene therapy to treat, inhibit or prevent diseases or disorders related to the abnormal expression and/or activity of the *Zip11.* The gene therapy refers to a therapy performed by administering an expressed or expressible nucleic acid to a subject. The nucleic acid molecule described in the present application produces a protein encoded thereby to achieve an effect of mediating the therapy. For example, after the encoding nucleic acid molecule or the expression product thereof is administered to a subject in need thereof, the activity and/or expression quantity of the *Zip11* may be increased.

The present application provides a method for identifying an aging condition in a subject. The method comprises determining an activity and/or expression quantity of the *Zip11* in the subject by using a substance capable of determining the activity and/or expression of the *Zip11* or an agent prepared from the substance, comparing the activity and/or expression quantity of the *Zip11* with a standard activity and/or expression quantity, which results in an increase or a decrease in the activity and/or expression quantity of the *Zip11* gene. In some cases, the method may be used for diagnosis/physical examination. For example, medical staff can judge an aging condition in a subject based on the activity and/or expression quantity of the *Zip11* in the subject. In some cases, the method may be used for researches, for example, to evaluate the effect of a candidate substances on the aging condition in the subject. In some cases, the method may also be used in a pharmaceutical process, for example, to identify and screen of a substance that affects aging. In some cases, the method may also be used in a diagnostic/physical examination process, for example, to identify the aging condition in the subject.

The present application provides use of a substance determining an activity and/or expression quantity of *Zip11* for the preparation of a reagent for identifying an aging condition in a subject. In some cases, an agent capable of detecting the expression quantity/activity of the *Zip11* may be prepared for use in diagnosis and/or physical examination.

The present application provides a system for identifying an aging condition in a subject. The system comprises a substance capable of determining an expression and/or activity of *Zip11.* In some cases, the system may be provided, sold, and/or promoted to related personnel, comprising healthcare suppliers, physicians, nurses, pharmacists, prescribers, drug developers, drug manufacturers, etc. In some other cases, the system may also be sold directly to consumers.

The present application further provides a method for assessing an effect of a candidate substance on an aging condition in a subject. In some cases, the method may be used in drug screening. For example, the candidate substance to be assessed is administered to the subject, and changes in the expression and/or activity of the *Zip11* in the subject are compared before and after the administration. If the expression and/or activity of the *Zip11* is increased, the candidate substance may be applied to the preparation of anti-aging drugs as a substance capable of delaying or ameliorating aging. In some other cases, the method may be used to evaluate the safety of the candidate substance. For example, the candidate substance to be assessed is administered to the subject, and changes in the expression and/or activity of the *Zip11* in the subject are compared before and after the administration. If the expression and/or activity of the *Zip11* is decreased, the substance is then a substance with toxic or side effects of accelerating or deteriorating the aging.

### Non-human animal or a part thereof

The present application provides a non-human animal or a part thereof, in which *Zip11* is overexpressed. In the present application, the term "overexpression" generally refers to the transcription or translation of a gene on a corresponding cell at a detectable level higher than that of normal cells of the same tissue type. Therefore, the overexpression may refer to the overexpression of proteins and RNAs (due to increased transcription, post-transcriptional processing, translation, post-translational processing, altered stability, and altered protein degradation), as well as local overexpression (increased nuclear localization) and enhanced functional activity caused by changes in protein transport patterns. For example, compared with normal cells or control cells, the overexpression may refer to an increase in the expression quantity by 100%, 150%, 200%, 250%, 300%, 400%, 500% or more. The overexpression may be achieved by methods known in the art. For example, it can be achieved by means of introducing exogenous genes. For example, a vector containing a promoter may be used to introduce the *Zip11* gene into the genome of a non-human animal for overexpression. The promoter may be a strong CAG promoter. The vector is able to insert a target gene into a target gene region in a host through homologous recombination. It may be any vector, such as a plasmid vector, a viral vector, a cosmid vector, a bacterial artificial chromosome (BAC), a yeast artificial chromosome (YAC) and other non-plasmid vectors, which are available in genetic engineering. In some cases, sgRNA targeting an H11 site in a mouse may be constructed by means of a Cas9/RNA system gene targeting technology. In the present application, the term "H11" generally refers to a ubiquitous locus that allows a strong exogenous promoter to be inserted therein to drive higher expression. In some other cases, a vector carrying both CAG-Loxp-Stop-Loxp-hZ/P7J-P2A-tdTomato fragments may be produced, and injected together with Cas9. After Cas9 cuts a DNA strand, the CAG-Loxp-Stop-Loxp-hZIP*11*- P2A-tdTomato fragments may be recombined to a target site by homologous recombination. A host cell which is knocked in the vector is introduced as a cell of the non-human animal or as a cell (comprising a cell cluster) differentiable into the cell of the non-human animal. As such a host cell, depending on the purpose, various cells may be used, and may be enumerated as, for example, ES cells, pluripotent stem cells such as iPS cells, germ-line stem cells such as sperm stem cells that are differentiable into germ cells, and fertilized eggs. The introducing of the knock-in vector into the host cell may be performed by a known method such as electroporation. The pluripotent stem cells may be injected into an early embryo by well-known methods such as microinjection, and the embryo is then transplanted into a surrogate mother to develop these stems cells, thereby obtaining chimeric animals. In addition, by breeding the chimeric animal, an individual with a homozygous knock-in array may be obtained from its offspring.

The non-human animal (for example, a *Zip11* KI mouse) or a part thereof, in which the *Zip11* gene is overexpressed, as described in the present application shows increased activity and/or expression quantity of the *Zip11,* increased sensitivity to immune stimulation (for example, in a concanavalin-induced liver injury, the contents of alanine aminotransferase and aspartate aminotransferase in the *Zip11* KI mice increase by at least 4 times, for example, 4 times, 5 times, 6 times or more, as compared with that of a wild type), and/or higher resistance to aging-related diseases. A tumor in the non-human animal or a part thereof, in which the *Zip11* gene is overexpressed, may be reduce or slowed down. For example, with the same tumor, a female *Zip11⁻* ^{^{/-}} mouse has a tumor volume larger than that of the wild type.

The present application further provides a non-human animal or a part thereof, which may be rodent or fish. For example, the non-human animal may be rodent. In the non-human animal or the part thereof, an expression and/or function of a *Zip11* gene is disrupted. In the present application, the term "disrupted" generally refers to the situation that a gene does not express a product and/or loses the function of expressing a product. This may consist in that the gene is structurally disrupted (for example, inserted with an Flp/FRT element or Cre/Loxp element), and consequently contains at least one mutation or structural change, such that the disrupted gene is not expressible or cannot direct the effective expression of an expression product. The destruction may be implemented by knocking out at least part of the *Zip11* gene. The gene knockout may be constructed by any method known in the art.

In some cases, it may be constructed with the aid of a Crisper/Cas9 system. Specifically, a *Zip11* allele mouse with a Flox site may be hybridized with a tool mouse that specifically expresses Cre to obtain a mouse model in which the expression and/or function of the *Zip11* gene is disrupted. In some other cases, a target gene may be knocked out by the principle of homologous recombination. For example, embryonic stem cell targeting may be used to modify an Slc39a11 gene with flox. In some cases, an exon 5 of the *Zip11* gene is at least partially knocked out. For example, the non-human animal or the part thereof may be a mouse model in which the *Zip11* gene is knocked out. The destruction means that the activity and/or expression quantity of the *Zip11* is significantly down-regulated compared with that of the wild type in which the *Zip11* gene is not knocked out.

The non-human animal or the part thereof, in which the expression and/or function of the *Zip11* gene is disrupted, as described in the present application has a decreased activity and/or expression quantity of the *Zip11,* showing a phenotype accelerating or deteriorating the aging. For example, in a *Zip11* KO zebrafish model, the reproduction rate of fertilized eggs in the females at the age of 6-12 months is reduced by about 10% to about 100% (for example, about 20% to about 90%, about 30% to about 80%, about 40% to about 70%, or about 50% to about 60%); the reproduction rate of fertilized eggs in the male zebrafish at the age of 0-3 months is reduced by about 10% to about 100% (for example, about 20% to about 80%, about 30% to about 70%, about 30% to about 60%, or about 40% to about 50%); and the reproduction rate of the males at the age of 6-12 months is lower than 1%. For another example, compared with the wild type, the survival rate of females at the age of 10 months among the *Zip11* KO zebrafish is 85%, and the survival rate of males is only 25%. For another example, the exercise capacity of a mouse with the *Zip11* knocked-out may be reduced by at least 30% (for example, by at least 40%, by at least 50%, by at least 60%, by at least 70%, by at least 80%, by at least 90% or more).

In some cases, the non-human animal or the part may be used for researches, for example, to evaluate the effect of a candidate substances on the activity and/or expression level of the *Zip11.* In some cases, the non-human animal or the part may be used in a pharmaceutical process, for example, to identify and screen substances that affect the activity and/or expression quantity of the *Zip11.* In some cases, the non-human animal or the part may be used in a diagnostic and/or physical examination process, for example, to identify an aging condition in a subject. In some cases, the non-human animal or the part may be provided, sold and/or promoted to related personnel, which comprise researchers, sales personnel, pharmaceutical personnel, etc.

In the present application, the term "comprise/comprise" generally refers to the inclusion of explicitly specified features, but not excluding other elements.

In the present application, the term "about" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

Not wishing to be bound by any particular theory, the following examples are merely to illustrate the fusion protein, preparation methods and uses and the like according to the present application, and are not intended to limit the scope of the present invention.

### Examples

### Example 1. Construction of zebrafish Zip11 gene knockout (KO) model

For the zebrafish gene (see NCBI database under Gene ID: 101882215 for the sequence), sgRNA was designed with a sequence was as set forth in SEQ ID NO: 4. pT7-gRNA plasmids as templates were subjected to PCR, and then recovered and purified from gel slices to obtain gRNA in vitro transcription templates, which were transcribed in vitro by using a T7 kit. 1% agarose gel electrophoresis was performed to confirm that sgRNAs were transcribed. The sgRNAs were then extracted and purified with phenol/chloroform/isoamyl alcohol to obtain a gRNA stock solution that was applicable to microinjection. pXT7-Cas9 plasmids were linearized with a restriction enzyme XbaI. After the agarose gel electrophoresis was performed to confirm that the linearization was complete, the linearized pXT7-Cas9 plasmids were purified through the column to obtain Cas9 mRNA in vitro transcription templates, which were transcribed in vitro by using the T7 kit.

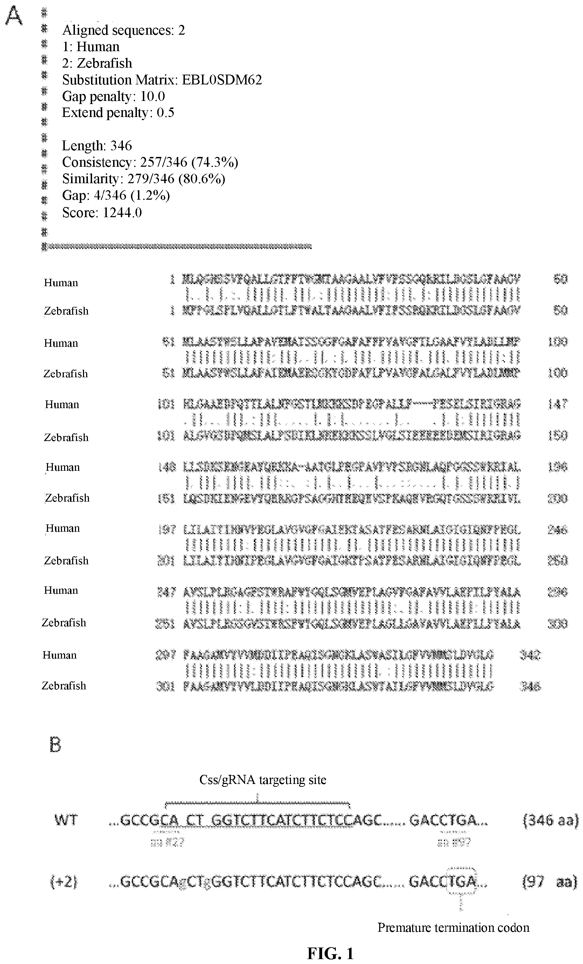

After the agarose gel electrophoresis was performed to confirm that gRNAs were transcribed, the gRNAs were purified with the kit to obtain a Cas9 mRNA stock solution that was applicable to microinjection. The sgRNAs and the Cas9-mRNAs were evenly mixed at an appropriate concentration, and were microinjected to zebrafish fertilized eggs, with uninjected wild-type zebrafish embryos as controls. The fertilized eggs were incubated and cultivated to obtain F0-generation Zip gene knockout zebrafish. FIG. 1A shows the alignment results of amino acid sequences of *Zip11* in human and zebrafish; and FIG. 1B shows the location of a Cas/gRNA target site for knocking out the zebrafish *Zip11* gene.

### Example 2. Aging condition phenotype of Zip11 KO zebrafish model

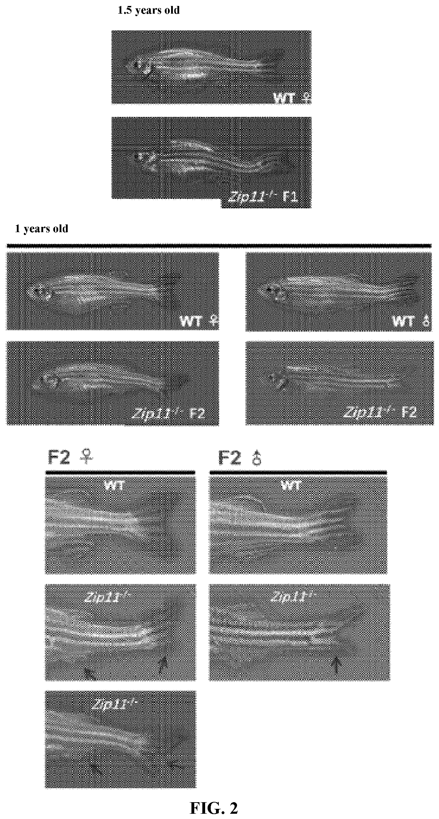

The F0-generation female and male zebrafish mated to produce F1-generation zebrafish homozygotes *(Zip11*^{*-*/}*⁻)*, and the F1-generation zebrafish mated to produce F2-generation *Zip11*^{*-*/*-*} mutants, which were incubated and cultured and then observed to record phenotypes. The results are as shown in FIG. 2. By continuously comparing and observing the *Zip11* knockout zebrafish and wild-type zebrafish, it was found that the 1.5-year-old F1-generation female zebrafish showed spinal curvature. The F2-generation female and male zebrafish showed spinal curvature with thin and degraded fins, and the wild-type zebrafish had intact fins.

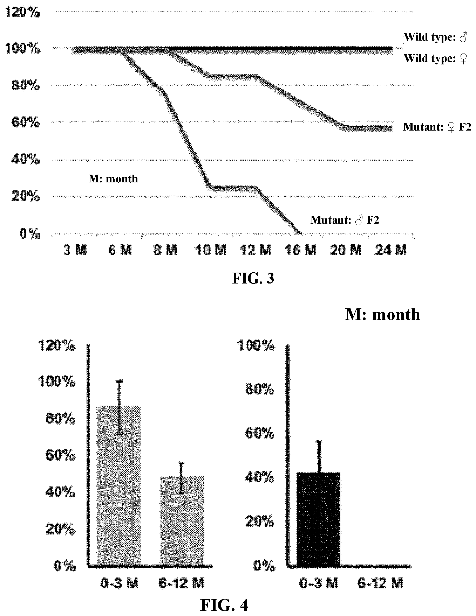

The survival conditions of F2-generation and wild-type zebrafish aged 3-24 months were recorded to plot survival curves. As shown in FIG. 3, the survival rate of the F2-generation *Zip11*^{*-*/*-*} zebrafish from birth to the age of 6 months was similar to that of the wild-type zebrafish; and from the age of 6 months, the male F2-generation zebrafish showed a higher mortality rate, and all died at the age of 16 months. Death occurred to the female F2-generation zebrafish at the age of 8 months, and after 20 months of age, their survival rate was less than 60%. It can be seen that the survival rate of female *Zip11*^{*-*/*-*} generation was higher than that of the male.

The eggs or sperms of F2-generation female and male zebrafish aged different months (0-3 months, 6-12 months) were fertilized *in vitro* with the eggs or sperms of wild-type zebrafish, and then incubated and cultured. The proportion of fertilized eggs developed into complete individuals was counted, with the result shown in FIG. 4. FIG. 4 shows that with the increase in the age of months, the proportion of fertile fertilized eggs decreases, and the proportion of fertile fertilized eggs produced by male F2-genration zebrafish was lower than that of the females.

FIGs. 2 to 4 show that the zebrafish lacking *Zip11* exhibit an aging phenotype, comprising spinal curvature, cachexia, regenerative decline, premature infertility and shortened lifespan, and the aging condition is more serious among the males.

### Example 3. Construction of Zip11 knockout (KO) mouse model

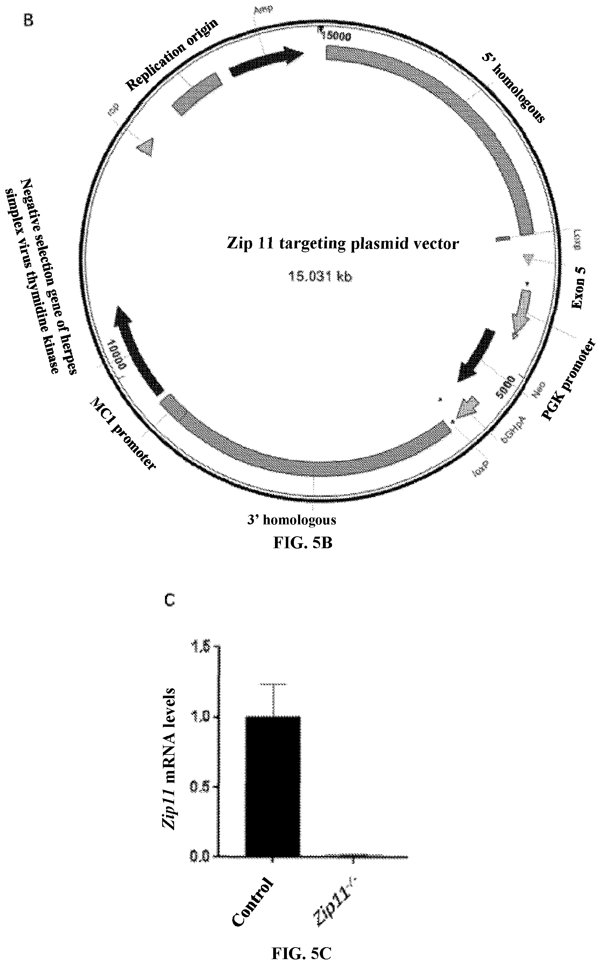

A *Zip11* KO mouse model was constructed based on the principle of homologous recombination. A target gene can be found under the MGI No.: 1917056 or the Ensembl No.: ENSMUSG00000041654 specific to a transcription version of ENSMUST00000071539. A targeting vector of embryonic stem cells was constructed by the Infusion method, which comprised 3.4kb5' homologous arms (5' homologous arm forward primer SEQ ID NO: 5, 5' homologous arm reverse primer SEQ ID NO: 9, 3' homologous arm forward primer SEQ ID NO: 9, and 3' homologous arm reverse primer SEQ ID NO: 10), a 2.3kb Flox region (SEQ ID NO: 13), PGK-Neo-polyA (SEQ ID NO: 14), 3.3kb 3' homologous arm, and an MC1-TK-polyA (SEQ ID NO: 15) negative selection marker, in which Flox targets an exon 5. FIG. 5B shows the plasmid profile of the targeting vector. After the vector was linearized, the vector was introduced into Jm8a3 embryonic stem cells by an electroporator. After G418 and Ganc drug screening, resistant clones were obtained; and after the long-fragment PCR identification, positive clones of homologous recombination were screened out.

The clones of positive embryonic stem cells were amplified, and then injected into the blastocysts of C57BL/6 mice. The injected blastocysts were transferred to the fallopian tubes of pseudo-pregnant female mice (C57BL/6J) according to standard procedures. After birth, chimeric mice were judged in terms of chimera and chimera degree based on the color of fur to obtain highly chimeric mice.

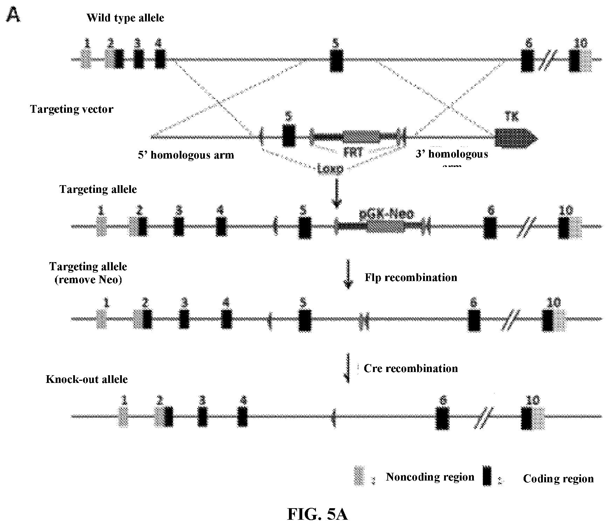

The chimeric male mice mated with Flp mice to breed F1-generation mice. Through PCR identification and sequencing confirmation, positive Neo heterozygous mice were obtained. The positive F1-generation heterozygous mice continued to mate with the Flp mice to breed F2-generation mice. Through PCR identification and sequencing confirmation, positive de-Neo heterozygous mice were obtained. Through Flp-mediated homologous recombination, target mice containing Flox sites at both ends of ***Zip11*** CKO (Conditional Knockout) were obtained, namely, ***Zip11*** allele mice with Flox sites. After the ***Zip11*** allele mice with Flox sites mated with tool mice in which Cre recombinase was specifically expressed, flox regions in their offspring of Flox homozygous Cre-positive mice were knocked out, resulting in functional deficiency of the target ***Zip11*** gene in specific tissue and cell types. A construction process was shown in FIG. 5A, and FIG. 5C showed that the expression level of *Zip11* in KO mice decreased in such a way that mRNA was scarcely transcribed, which indicated that the knockout was successful.

### Example 4. Phenotypes of aging conditions in Zip11 KO mice

### 4.1 Hair loss symptoms occurred to female and male Zip11 KO mice

The mice had been kept in an SPF-grade animal house. After weaning from the age of 22 days, the mice were caged in terms of gender, and ate and drunk at will. The animal house was maintained at a temperature of 22±3°C and a humidity of 35±5%, with a 12-hour day/night cycle. The apparent changes of the mice were observed on a regular basis.

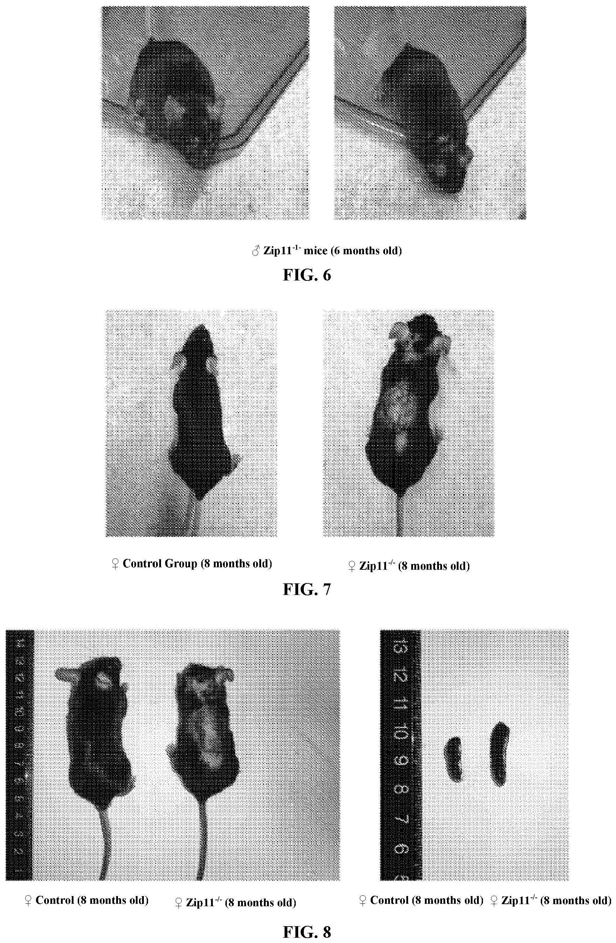

From FIG. 6, it can be seen that the male *Zip11*^{*-*/*-*} mice show a hair loss symptom on their heads at the age of 6 months. FIG. 7 shows that the female *Zip11* KO mice show a hair loss symptom on their backs and head at the age of 8 months.

### 4.2 Spleen enlargement symptoms occurred to Zip11 mice

When the mice were 8-month-old, the Zip11 knockout mice and the wild-type mice were anesthetized and sacrificed. Their spleens and livers were quickly removed, fixed with 4% paraformaldehyde for 48 hours, embedded in paraffin, and sectioned into 5µm tissue slices, which were stained with hematoxylin-eosin (H&E) as usual and mounted with neutral balsam, and were observed under a microscope for histological changes.

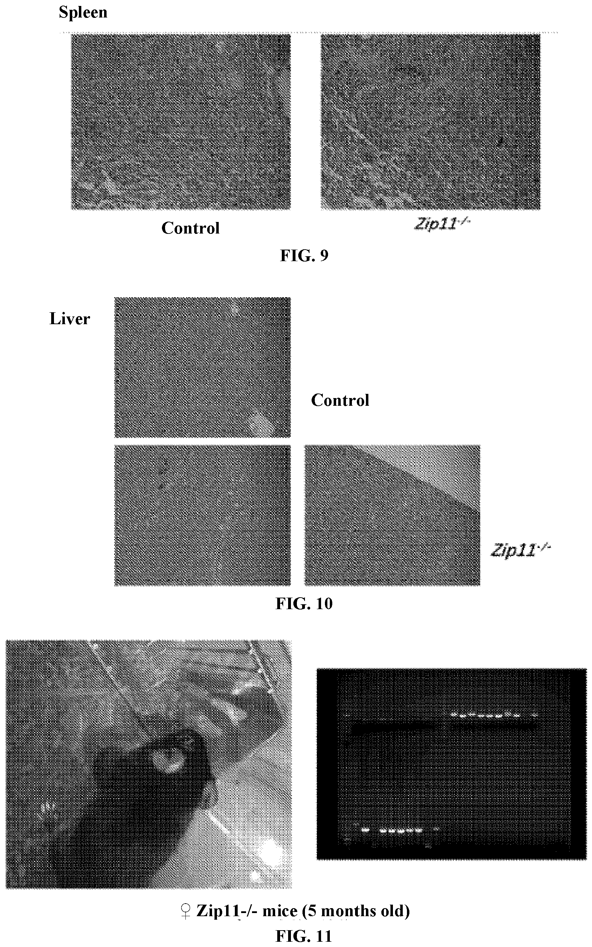

FIG. 8 shows spleen enlargement symptoms occurred to the female *Zip11 KO* mice at the age of 8 months. The spleens of these mice were enlarged compared with those of the wide-type mice in the control group. The spleen slices showed that the spleen tissues of *Zip11* gene knockout mice was enlarged (FIG. 9).

### 4.3 Liver enlargement symptoms occurred to Zip11 KO mice

The livers of the mice were sectioned and observed according to the above method. It was found that the liver tissues of *Zip11K*O mice increased in volume (FIG. 10).

### 4.4 Spontaneous neogenesis occurred to Zip11 KO mice

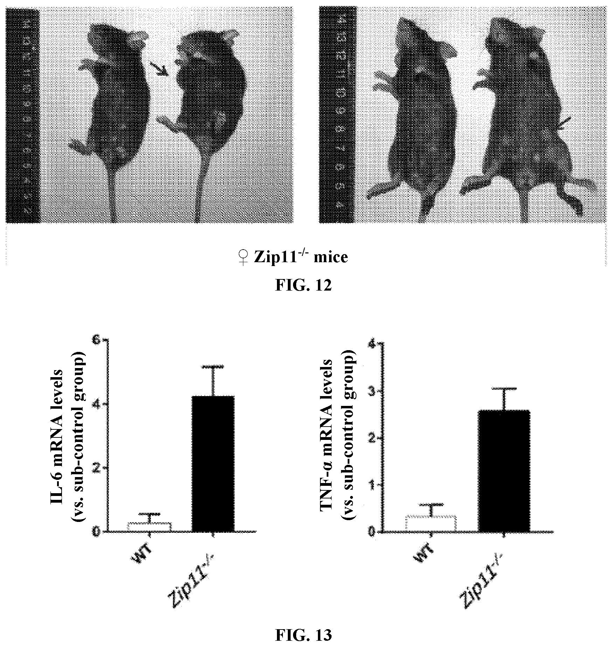

FIG. 11 shows a photo of a laboratory mouse on the left, and an electrophoretogram of the genotype of the female laboratory mouse on the right, which indicates that the mouse was a *Zip11* gene knockout mouse. When this *Zip11* gene knockout mice were 5-month-old, a mass was observed on the left forelimb of the mouse; and in addition, a breast mass was also observed in the mouse. The wild-type control mice in the same cage did not show the phenotype of spontaneous tumor growth (FIG. 12). From FIG. 12, it can be seen that the *Zip11* KO mice show obvious tumor growth.

### 4.5 Higher levels of inflammatory factors in Zip11 KO mice

Compared with the wild-type mice, the RT-PCR test results (FIG. 13) of the liver tissues of *Zip11*^{*-*/-} mice showed that the mRNA expression levels of inflammatory factors such as IL-6 and TNF-α in *Zip11*-deficient mice were increased, indicating a stronger inflammatory response.

### 4.6 Decreased exercise capacity in Zip11 KO mice

The effect of *Zip11* on the exercise capacity was evaluated by using a linear acceleration rotarod (Panlab/Harvard Apparatus, Holliston, USA) test. The *Zip11 KO* mice and the wild-type mice were placed on the linear acceleration rotarod. The speed was increased from 4 rpm to 40 rpm over a period of 300 seconds. An acceleration scheme was used to eliminate the need for adaptation to the rotarod, thereby minimizing the divergence of the results obtained from each mouse due to disproportionate performance improvements. Each test was ended once the mouse fell from the rotarod, wherein 3 rounds of accelerated rotarod operation was performed for each mouse on each experimental day, and the test was performed for 4 experimental days. The animals were allowed to recover for 5 minutes between tests, in order to prevent performance decrease induced by fatigue. The *Zip11 KO* mice and the wild-type mice were compared in terms of the time when they fell from the rotarod.

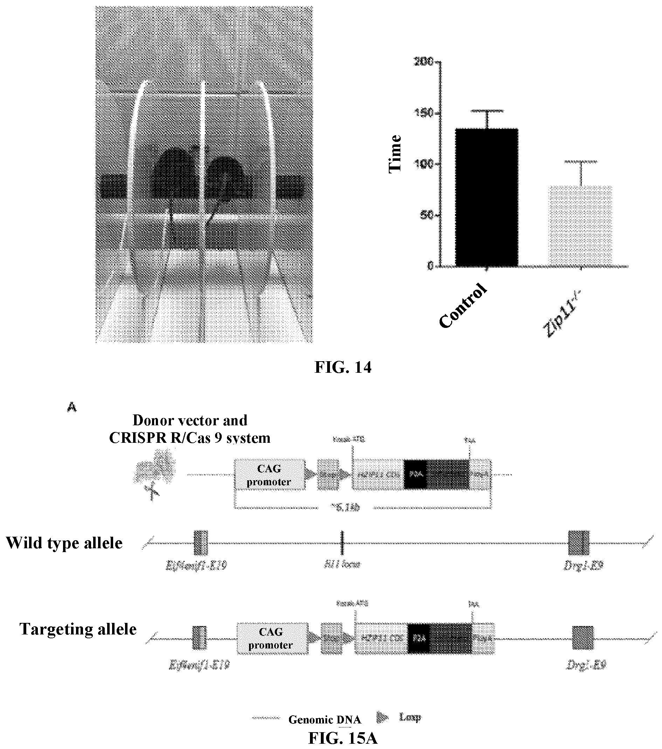

The results were shown in FIG. 14, in which the time taken by the Zip11 KO mice to maintain rotation was shorter than that of the wild-type mice, indicating weaker exercise capacity.

### Example 5. Obesity in male Zip11 KO mice

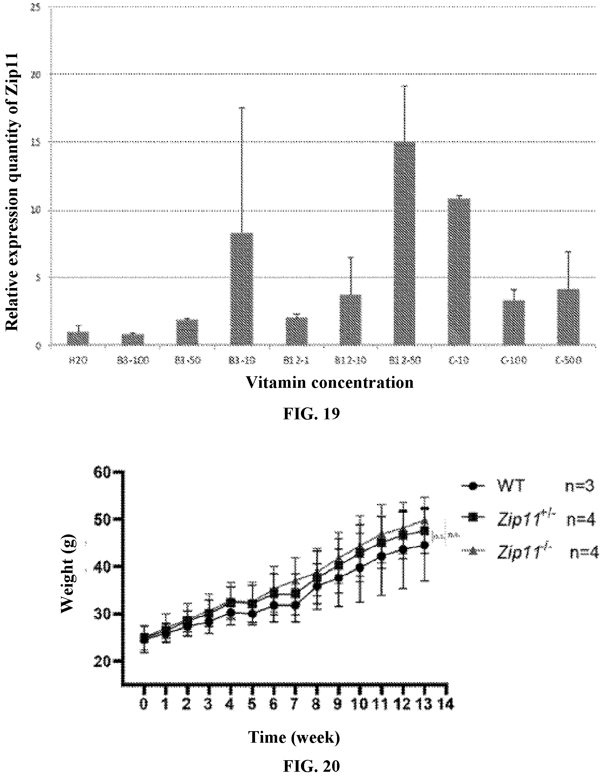

The male *Zip11* KO mice and the wild-type mice, comprising 3 wild-type mice, 4 *Zip11*^{+/-} mice, and 4 *Zip11*^{*-*/*-*} mice, were fed with a high-fat diet (HFD) to construct a high-fat model. Their body weights were measured every week. The results were shown in FIG. 20. Compared with the wild-type mice, the weight of the male *Zip*11 mice was slightly heavier, indicating that the knockout in the male mice would lead to obesity in the male mice.

### Example 6. Impaired cognitive and learning capacities in Zip11 KO mice

A high-fat model of the *Zip11* knockout mice was built over 7 weeks. Then, a series of investigations on behavioral capacity were carried out on the mice (4-5 months old) in an open field test. In this test, the phobotaxis of the mice was utilized. That is, the mice were afraid of open and unfamiliar environments, and thus tend to move in a peripheral area in an open field. The mice also had the nature to explore new environments and thus would explore in a central area. If the mice moved in the central area for a long time over a long distance, it indicated that their desire to explore and cognitive capacity were high. On the contrary, if the mice moved in the central area for a short time over a short distance, it indicated that their desire to explore and cognitive capacity were low.

Specifically, each of the gene-knockout male mice (4 (*Zip11*^{+/-} mice, 4 *Zip11*^{*-*/*-*} mice) and the wild-type male mice (3) was placed right in the middle of an open field test device, and was allowed to move freely in the test device for 15 minutes. The movement distances in the peripheral and central areas of the open field, as well as the total distance and the time were recorded for the mice. After each mouse underwent the test, the test device was wiped with 75% alcohol, and the feces and urine of the mouse was cleared away, in order to prevent the odor left by the previous mouse from affecting the behavior of the next mouse.

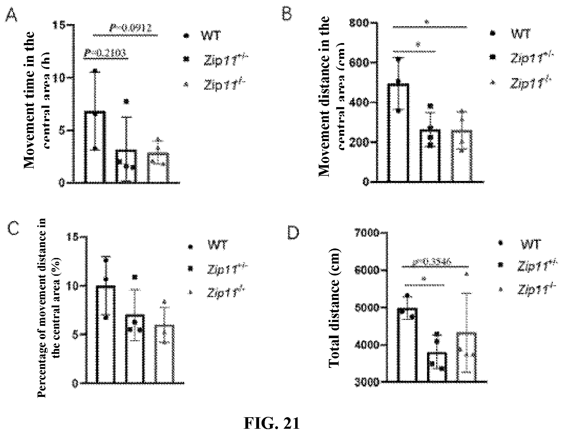

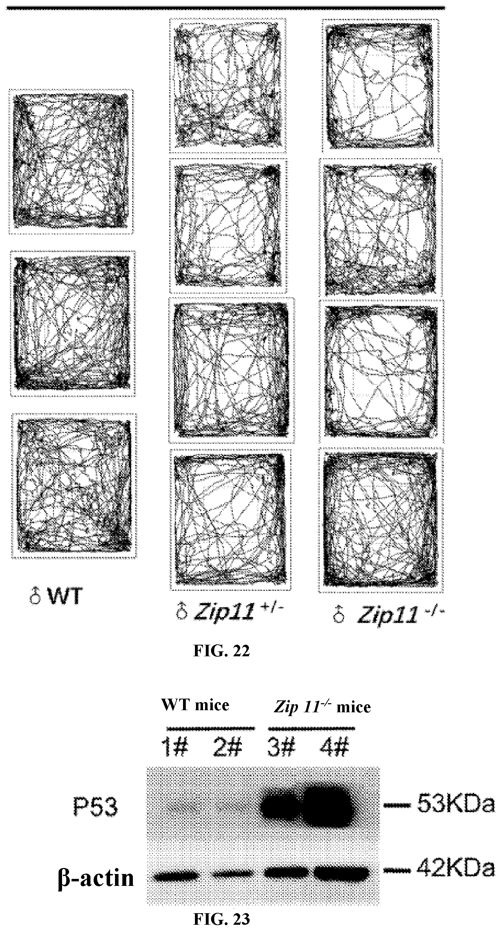

The statistical results of the open field test were shown in FIG. 21, and the movement trajectories of the mice were shown in FIG. 22. The movement distance and time of the Zip11 knockout mouse in the central area somewhat decreased, indicating that the learning and cognitive capacities would be affected, and the desire to explore and cognitive capacity decreased.

### Example 7. Increased p53 protein level in Zip11 KO mice

The female and male *Zip11* knockout mice of 8 months old and their corresponding wild-type mice were anesthetized. Their tail tissues were quickly cut, and extracted for the protein in mouse tissue cells. Cell debris and other impurities were removed. The p53 protein expression level was detected by Western blot. It was found that the p53 level of the gene knockout mice increased significantly (FIG. 23), indicating the association between the *Zip11* gene knockout and the aging with increased p53.

### Example 8. Weakened immune function of macrophages in Zip11 KO mice under the stimulation of LPS

Acute inflammation was induced in wild-type (WT) mice (n=6) and Zip11 KO mice (n=4) by intraperitoneal injection of LPS (2.5 mg/kg) and D-galactosamine (0.25 g/kg). After 6 hours, the mice were anesthetized and dissected. The blood and spleen were taken, and added with labeled anti-CD86 antibodies and labeled CD206 antibodies, respectively. A ratio of keto CD86-positive (M1 type macrophages) cells to keto CD206-positive (M2 type macrophages) cells was detected by flow cytometry.

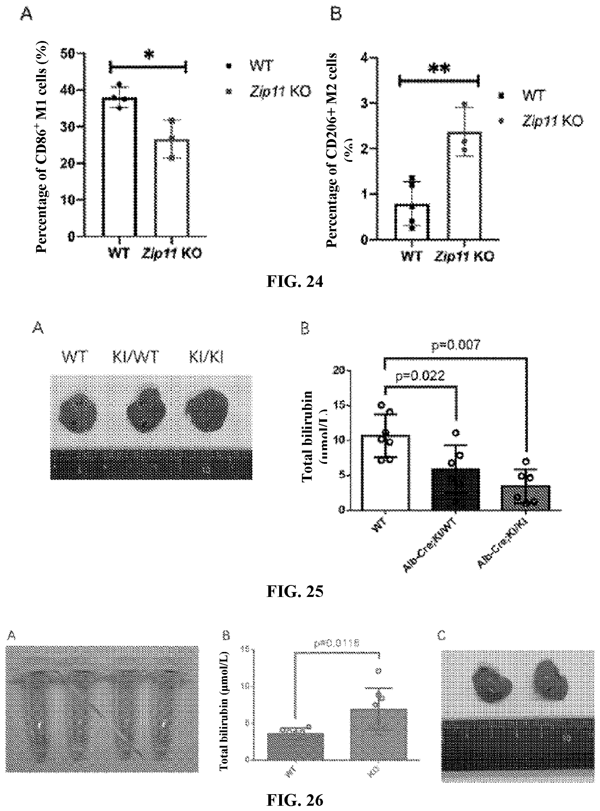

The M1 type macrophages exhibited strong pro-inflammatory and antigen-presenting capabilities, and exerted host immune clearance functions against pathogens and tumor cells. The M2 type macrophages had effects such as resisting inflammation, promoting wound healing and fibrosis, repairing tissues, and promoting tumor growth and infiltration. The results showed that after the mice were stimulated with LPS, the M1 macrophages were significantly reduced (FIG. 24A), and the M2 macrophages were significantly increased, indicating a weakened immune function of the macrophages (FIG. 24B).

### Example 9. Construction of ZIP11 knock-in mouse model

sgRNA (with a sequence from 5' to 3' as set forth in SEQ ID NO: 2) and PAM (with a sequence as set forth in SEQ ID NO: 3) sequences targeting the mouse H11 site were constructed. Corresponding donors were designed based on sgRNA to construct a targeting plasmid vector containing the *ZIP11* coding region. The vector also contains a strong CAG promoter and a termination signal with Loxp on both sides, and a tdTomato reporter gene was inserted to be linked to human *ZIP 11* via a 2A short peptide. FIG. 15A shows a process of constructing a *ZIP11 KI* mouse model. The coding region sequence of human *ZIP11 (hZIP11)* was as set forth in SEQ ID NO: 1.

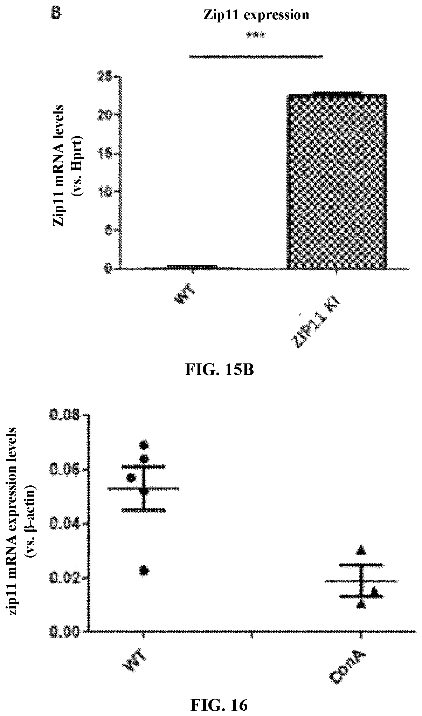

Then, the vector was injected into C57BL/6J embryonic stem cells by microinjection. Then, the blastocysts were transferred to pseudo-pregnant C57BL/6J female mice to obtain chimeric mice. The mice born by microinjection were subjected to PCR, sequencing and southern blot identification, and positive F0 mice were obtained. The F0 mice were bred with C57BL/6J respectively, and the PCR, sequencing and southern identification were carried out to obtain positive F1. Furthermore, the homozygous gene knockin mice obtained by mating heterozygous mice were bred with tool mice specifically expressing Cre recombinase to obtain *H11-ZIP11* knockin (KI) mice. After the mice were anesthetized and sacrificed, the livers of the *ZIP11* KI mice were quickly removed to detect the *ZIP* mRNA expression level. FIG. 15B shows that, compared with the wild type, the liver parenchymal cells of the *ZIP11* KI mice specifically overexpress ZIP11, indicating successful knockin.

### Example 10. Decreased expression level of Zip11 due to concanavalin A

The wild-type C57BL/6 mice were divided into two groups. An experimental group was injected with 10 mg/kg concanavalin (ConA) through the tail vein. After 18 hours, the liver tissues were removed, and the expression level of *ZIP11* was detected by RT-PCR, with β-actin as a reference. The results were found in FIG. 16, and ConA may decrease the level of *ZIP 11.*

### Example 11. High sensitivity in ZIP11 KI mice to liver inflammation indued by concanavalin A

The *ZIP11* KI mice of 8 weeks old and the wild-type control mice in the same cage were injected with 10 mg/kg concanavalin (ConA) through the tail vein. After 18 hours, the mice were anesthetized, and then sacrificed after cardiac puncture and serum collection. The liver tissues were removed, shot and weighed. The liver tissues were fixed with 4% paraformaldehyde, embedded in paraffin, and sectioned into slices, which were stained with H&E and TUNEL respectively, and then mounted to observe histological changes.

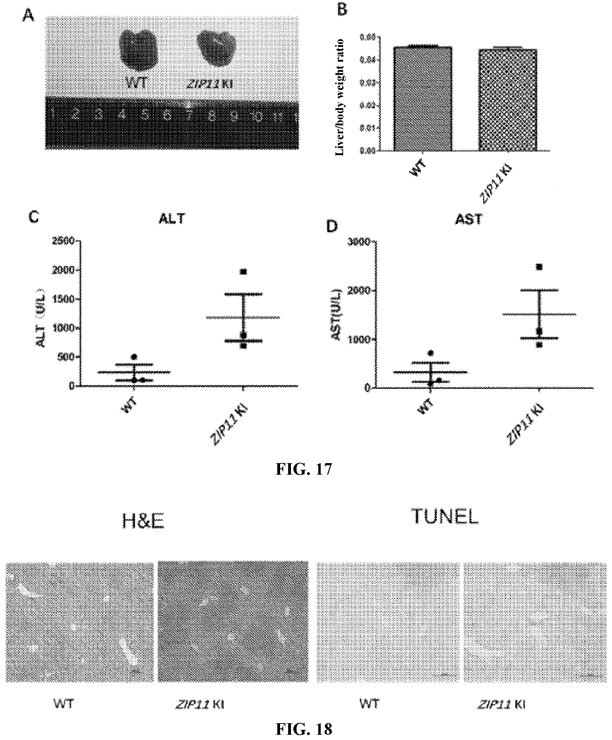

The levels of alanine aminotransferase (ALT) and aspartate aminotransferase (AST) in serum were detected. The results are as shown in FIGs. 17 and 18.

FIG. 17A shows the comparison in liver size as well as appearance and color between the wild-type and *ZIP* KI mice in the ConA-induced liver inflammation injury model. FIG. 17B shows a ratio of liver weight to body weight, and FIGs. 17C and 17D show increased serum ALT and AST levels in *ZIP11* KI mice. FIG. 18 shows the morphology of liver cells under different staining methods. These results show that the *ZIP11* KI mice were more sensitive to the liver injury induced by ConA.

### Example 12. Decreased bilirubin levels in ZIP11 KI male mice

A methionine and choline deficient L-amino acid diet (MCD, i.e., Methionine and Choline Deficient L-Amino Acid Diet, a dietary feed free of methionine and choline sources) was fed to male mice with *ZIP11* overexpressed in the liver, wide-type male mice of 8 weeks old, and *Zip 11* knockout mice, respectively. The mice were allowed free access to the food and drinking water to construct non-alcoholic fatty liver models (NASHs). After four weeks, the mice were anesthetized, dissected to remove the livers, which were shot and weighed for a series of physiology indicator tests.

Based on the results, FIG. 25A shows a liver comparison diagram of the wild-type mice and the male overexpressing mice. The serum bilirubin content was detected at the Center for Drug Safety Evaluation and Research of Zhejiang University. The experimental results showed that the serum bilirubin in the male mice with *ZIP11* overexpressed in the liver significantly decreased (FIG. 25B), and the serum bilirubin in the male *Zip11* knockout mice significantly increased compared with the wild-type mice (FIGs. 26A-26C). In summary, *ZIP11* plays a protective role in the NASH modeled mice fed with MCD.

### Example 13. Vitamins capable of increasing the expression level of Zip11

Human HEK293 and HT1080 cells were treated for 24 hours respectively at different concentrations of vitamin B3 (10 µM, 50 µM, 100 µM), vitamin B12 (1 µM, 10 µM, 50 µM) and vitamin C (10 µM, 100 µM, 500 µM). The relative expression quantity of *Zip11* mRNA was detected. The results were shown in FIG. 19. The results show that vitamins can increase the expression level of Zip11.

The foregoing detailed description is provided by way of explanation and examples, and is not intended to limit the scope of the appended claims. Various changes of the embodiments listed in the present application until now are obvious to those of ordinary skill in the art, and should be kept within the scope of the appended claims and equivalents thereof.

## Claims

1. A method for identifying a substance capable of affecting aging, comprising:
determining an effect of a candidate substance on an activity and/or expression of Zip11; and identifying said candidate substance, that changes said activity and/or expression of the Zip11, as said substance capable of affecting said aging.

2. The method according to Claim 1, wherein said determining the effect of said candidate substance on said activity and/or expression of the Zip11 comprises: determining an effect of said candidate substance on a nucleic acid expression level and/or activity of the Zip11.

3. The method according to Claim 2, wherein said nucleic acid expression level and/or activity of the Zip11 is determined by using a substance selected from the group consisting of: a primer capable of specifically amplifying the Zip11, and a nucleic acid probe capable of specifically recognizing the Zip11.

4. The method according to any one of Claims 1-3, wherein said determining the effect of said candidate substance on said activity and/or expression of the Zip11 comprises: determining the effect of said candidate substance on a protein expression level and/or activity of the Zip11.

5. The method according to Claim 4, wherein said protein expression level and/or activity of the Zip11 is determined by using a substance selected from the group consisting of: a reagent specifically recognizing a protein of the Zip11, and a substance capable of determining a protein activity of the Zip11.

6. The method according to any one of Claims 1-5, wherein a candidate substance that increases said activity and/or expression of the Zip11 is identified as a substance capable of delaying or ameliorating said aging.

7. The method according to any one of Claims 1-6, wherein a candidate substance that reduces said activity and/or expression of the Zip11 is identified as a substance capable of accelerating or deteriorating said aging.

8. The method according to any one of Claims 1-7, comprising administering said candidate substance to a non-human animal, an organ, a tissue and/or a cell, determining the effect of said candidate substance on said activity and/or expression of the Zip11 in said non-human animal, said organ, said tissue and/or said cell, and identifying said candidate substance, that changes said activity and/or expression of the Zip11, as said substance capable of affecting said aging.

9. The method according to any one of Claims 1-8, wherein the method is an *in vitro* or *ex vivo* method.

10. The method according to any one of Claims 1-9, wherein a condition of said aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

11. The method according to Claim 10, wherein said body physique condition is evaluated by detecting a spine form.

12. The method according to any one of Claims 10-11, wherein said hair condition is evaluated by detecting hair loss.

13. The method according to any one of Claims 10-12, wherein said immune level condition is evaluated by detecting a cytokine level.

14. The method according to any one of Claims 10-13, wherein said tumor-bearing condition is evaluated by detecting a tumor volume, tumor progression, and/or an occurrence of tumor cells.

15. The method according to any one of Claims 10-14, wherein said liver condition is evaluated by detecting an alanine aminotransferase activity, an aspartate aminotransferase activity, and /or a liver/body weight ratio.

16. The method according to any one of Claims 10-15, wherein said spleen condition is evaluated by detecting a spleen/body weight ratio.

17. The method according to any one of Claims 10-16, wherein said reproductive capacity condition is evaluated by detecting a proportion of fertile eggs.

18. The method according to any one of Claims 10-17, wherein said exercise capacity condition is evaluated from fatigue rotarod test results.

19. The method according to any one of Claims 10-18, wherein said lifespan condition is evaluated by detecting a survival time.

20. The method according to any one of Claims 10-19, wherein said cognitive capacity condition is evaluated by an open field test.

21. Use of a substance capable of increasing an activity and/or an expression quantity of Zip11 for the preparation of a drug for delaying or ameliorating aging.

22. The use according to Claim 21, wherein said delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, delaying a time point of exercise capacity degeneration, and/or increasing a behavioral cognitive capacity.

23. The use according to any one of Claims 21-22, wherein said increasing refers to an increase of at least 15 times in said activity and/or expression quantity of the Zip11 after administration of said substance as compared to that of without administration of said substance.

24. The use according to any one of Claims 21-23, wherein said activity and/or expression quantity of the Zip11 comprises an activity and/or expression quantity at a nucleic acid level of the Zip11 and/or at a protein level of the Zip11.

25. A system for identifying a substance capable of affecting aging, comprising a substance capable of determining an effect of a candidate substance on an activity and/or expression of Zip11.

26. The system according to Claim 25, comprising a substance capable of determining a nucleic acid expression level and/or activity of the Zip11.

27. The system according to Claim 26, wherein said substance capable of determining said nucleic acid expression level and/or activity of the Zip11 comprises: a primer capable of specifically amplifying the Zip11 and/or a probe capable of specifically recognizing the Zip11.

28. The system according to any one of Claims 26-27, comprising a substance capable of determining a protein expression level and/or activity of the Zip11.

29. The system according to Claim 28, wherein said substance capable of determining said protein expression level and/or activity of the Zip11 comprises a reagent capable of specifically recognizing a protein of the Zip11 and/or a reagent capable of determining a protein activity of the Zip11.

30. Use of a nucleic acid molecule encoding Zip11, or an expression product thereof, for the preparation of a drug for delaying or ameliorating aging.

31. The use according to Claim 30, wherein said delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, delaying a time point of exercise capacity degeneration, and/or increasing a behavioral cognitive capacity.

32. The use according to any one of Claims 30-31, wherein the nucleic acid molecule comprises a nucleotide sequence as set forth in SEQ ID No. 1.

33. A method for delaying or ameliorating aging, comprising administering an effective amount of a nucleic acid molecule encoding Zip11, or an expression product thereof, to a subject in need thereof.

34. The method according to Claim 33, wherein said delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, delaying a time point of exercise capacity degeneration, and/or increasing a behavioral cognitive capacity.

35. The method according to any one of Claims 33-34, wherein said nucleic acid molecule comprises a nucleotide sequence as set forth in SEQ ID No. 1.

36. A method of identifying an aging condition in a subject, comprising: determining an activity and/or expression quantity of Zip11 in said subject.

37. The method according to Claim 36, wherein said activity and/or expression quantity of the Zip11 comprises a nucleic acid expression level and/or activity of the Zip11, and/or a protein expression level and/or activity of the Zip11.

38. The method according to Claim 37, wherein said nucleic acid expression level and/or activity of the Zip11 in said subject is determined by using a substance selected from the group consisting of: a primer capable of specifically amplifying the Zip11, and a nucleic acid probe capable of specifically recognizing the Zip11.

39. The method according to any one of Claims 37-38, wherein said protein expression level and/or activity of the Zip11 in said subject is determined by using a substance selected from the group consisting of: a reagent specifically recognizing a protein of the Zip11, and a substance capable of determining a protein activity of the Zip11.

40. The method according to any one of Claims 36-39, wherein a condition of said aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

41. The method according to Claim 40, wherein said body physique condition is evaluated by detecting a spine form.

42. The method according to any one of Claims 40-41, wherein said hair condition is evaluated by detecting hair loss.

43. The method according to any one of Claims 40-42, wherein said immune level condition is evaluated by detecting a cytokine level.

44. The method according to any one of Claims 40-43, wherein said tumor-bearing condition is evaluated by detecting a tumor volume, tumor progression, and/or an occurrence of tumor cells.

45. The method according to any one of Claims 40-44, wherein said liver condition is evaluated by detecting an alanine aminotransferase activity, an aspartate aminotransferase activity, and /or a liver/body weight ratio.

46. The method according to any one of Claims 40-45, wherein said spleen condition is evaluated by detecting a spleen/body weight ratio.

47. The method according to any one of Claims 40-46, wherein said reproductive capacity condition is evaluated by detecting a proportion of fertile eggs.

48. The method according to any one of Claims 40-47, wherein said exercise capacity condition is evaluated from fatigue rotarod test results.

49. The method according to any one of Claims 40-48, wherein said lifespan condition is evaluated by detecting a survival time.

50. The method according to any one of Claims 40-49, wherein said cognitive capacity condition is evaluated by an open field test.

51. Use of a substance determining an activity and/or expression quantity of Zip11 for the preparation of a reagent for identifying an aging condition in a subject.

52. The use according to Claim 51, wherein said activity and/or expression quantity of the Zip11 comprises a nucleic acid expression level and/or activity of the Zip11, and/or a protein expression level and/or activity of the Zip11.

53. The use according to any one of Claims 51-52, wherein said substance comprises: a primer capable of amplifying the Zip11, a nucleic acid probe capable of specifically recognizing the Zip11, a reagent capable of specifically recognizing a protein of the Zip11, and/or a substance capable of determining a protein activity of the Zip11.

54. The use according to any one of Claims 51-53, wherein a condition of said aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

55. The use according to Claim 54, wherein said body physique condition is evaluated by detecting a spine form.

56. The use according to any one of Claims 54-55, wherein said hair condition is evaluated by detecting hair loss.

57. The use according to any one of Claims 54-56, wherein said immune level condition is evaluated by detecting a cytokine level.

58. The use according to any one of Claims 54-57, wherein said tumor-bearing condition is evaluated by detecting a tumor volume, tumor progression, and/or an occurrence of tumor cells.

59. The use according to any one of Claims 54-58, wherein said liver condition is evaluated by detecting an alanine aminotransferase activity, an aspartate aminotransferase activity, and /or a liver/body weight ratio.

60. The use according to any one of Claims 54-59, wherein said spleen condition is evaluated by detecting a spleen/body weight ratio.

61. The use according to any one of Claims 54-60, wherein said reproductive capacity condition is evaluated by detecting a proportion of fertile eggs.

62. The use according to any one of Claims 54-61, wherein said exercise capacity condition is evaluated from fatigue rotarod test results.

63. The use according to any one of Claims 54-62, wherein said lifespan condition is evaluated by detecting a survival time.

64. The use according to any one of Claims 54-63, wherein said cognitive capacity condition is evaluated by an open field test.

65. A system for identifying an aging condition in a subject, comprising a substance capable of determining an expression and/or activity of Zip11.

66. The system according to Claim 65, comprising a substance capable of determining a nucleic acid expression level and/or activity of the Zip11.

67. The system according to Claim 66, wherein said substance capable of determining said nucleic acid expression level and/or activity of the Zip11 comprises: a primer capable of amplifying the Zip11 and/or a nucleic acid probe capable of specifically recognizing the Zip11.

68. The system according to any one of Claims 65-67, comprising a substance capable of determining a protein expression level and/or activity of the Zip11.

69. The system according to Claim 68, wherein said substance capable of determining said protein expression level and/or activity of the Zip11 comprises: a substance capable of specifically recognizing a protein of the Zip11 and/or a substance capable of determining a protein activity of the Zip11.

70. The system according to any one of Claims 65-69, wherein a condition of said aging is determined by evaluating one or more conditions selected from the group consisting of: a body physique condition, a hair condition, an immune level condition, a tumor-bearing condition, a liver condition, a spleen condition, a reproductive capacity condition, an exercise capacity condition, a lifespan condition, and a cognitive capacity condition.

71. The system according to Claim 70, wherein said body physique condition is evaluated by detecting a spine form.

72. The system according to any one of Claims 70-71, wherein said hair condition is evaluated by detecting hair loss.

73. The system according to any one of Claims 70-72, wherein said immune level condition is evaluated by detecting a cytokine level.

74. The system according to any one of Claims 70-73, wherein said tumor-bearing condition is evaluated by detecting a tumor volume.

75. The system according to any one of Claims 70-74, wherein said liver condition is evaluated by detecting an alanine aminotransferase activity, an aspartate aminotransferase activity and /or a liver/body weight ratio.

76. The system according to any one of Claims 70-75, wherein said spleen condition is evaluated by detecting a spleen/body weight ratio.

77. The system according to any one of Claims 70-76, wherein said reproductive capacity condition is evaluated by detecting a proportion of fertile eggs.

78. The system according to any one of Claims 70-77, wherein said exercise capacity condition is evaluated from fatigue rotarod test results.

79. The system according to any one of Claims 70-78, wherein said lifespan condition is evaluated by detecting a survival time.

80. The system according to any one of Claims 70-79, wherein said cognitive capacity condition is evaluated by an open field test.

81. A method for assessing an effect of a candidate substance on an aging condition in a subject, comprising:
1) administering said candidate substance to be assessed to said subject;
2) comparing changes in an expression and/or activity of Zip 11 in said subject before and after said administering; and
3) assessing said effect of said candidate substance on said aging condition in said subject based on said changes in said expression and/or activity of the Zip11.

82. The method according to Claim 81, wherein said activity and/or expression quantity of the Zip11 comprises an activity and/or expression quantity at a nucleic acid level of the Zip11 and/or at a protein level of the Zip11.

83. The method according to any one of Claims 81-82, wherein said candidate substance delays or ameliorates said aging condition in said subject in the case of up-regulated expression and/or increased activity of the Zip11 in said subject after said administrating.

84. The method according to Claim 83, wherein said delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, increasing a behavioral cognitive capacity, and/or delaying a time point of exercise capacity degeneration.

85. The method according to any one of Claims 81-84, wherein said candidate substance accelerates or deteriorates said aging condition in said subject in the case of down-regulated expression and/or reduced activity of the Zip11 in said subject after said administrating.

86. The method according to Claim 85, wherein said accelerating or deteriorating the aging comprises: exacerbating spinal curvature, exacerbating hair loss, decreasing a cytokine level, weakening immunity, deteriorating or recurring tumors, shortening a lifespan, exacerbating a liver injury, exacerbating a spleen injury, exacerbating decreased fertility, reducing an exercise capacity, and/or reducing a behavioral cognitive capacity.

87. A non-human animal or a part thereof, in which Zip11 is over-expressed.

88. The non-human animal or the part thereof according to Claim 87, comprising an additional number of copies of a Zip11 gene as compared to a corresponding wild type.

89. The non-human animal or the part thereof according to Claim 88, wherein an expression of said Zip11 gene is regulated by a tissue-specific promoter.

90. The non-human animal or the part thereof according to any one of Claims 88-89, wherein said additional number of copies of said Zip11 gene are specifically expressed in the liver.

91. The non-human animal or the part thereof according to any one of Claims 89-90, wherein said tissue-specific promoter comprises a nucleotide sequence as set forth in SEQ ID No. 1.

92. The non-human animal or the part thereof according to any one of Claims 87-91, which shows a phenotype delaying or ameliorating an aging condition.

93. The non-human animal or the part thereof according to Claim 92, wherein said delaying or ameliorating the aging comprises: preventing or treating spinal curvature, preventing or treating hair loss, increasing a cytokine level, enhancing immunity, preventing or treating tumors, prolonging life, preventing or treating a liver injury, preventing or treating a spleen injury, preventing or treating decreased fertility, improving an exercise capacity, delaying a time point of exercise capacity degeneration, and/or increasing a behavioral cognitive capacity.

94. A non-human animal or a part thereof, in which an expression and/or function of a Zip11 gene is disrupted.

95. The non-human animal or the part thereof according to Claim 94, wherein said Zip11 gene is at least partially knocked out.

96. The non-human animal or the part thereof according to Claim 95, wherein an exon 5 of said Zip11 gene is at least partially knocked out.

97. The non-human animal or the part thereof according to any one of Claims 94-96, which shows a phenotype accelerating or deteriorating an aging condition.

98. The non-human animal or the part thereof according to any one of Claims 94-97, wherein said accelerating or deteriorating the aging comprises: exacerbating spinal curvature, exacerbating hair loss, decreasing a cytokine level, weakening immunity, deteriorating or recurring tumors, shortening a lifespan, exacerbating a liver injury, exacerbating a spleen injury, exacerbating decreased fertility, reducing an exercise capacity, and/or reducing a behavioral cognitive capacity.


SEQUENCE LISTING

<110>  ZHEJIANG BAISHOUKANGDI CO. LTD.

<120>  METHOD FOR IDENTIFYING SUBSTANCE CAPABLE OF AFFECTING AGING¡¡

<130>  0121-PA-002EP

<160>  15

<170>  PatentIn version 3.5

<210>  1
<211>  1026
<212>  DNA
<213>  Artificial Sequence

<220>
<223>  hZIP11 CDS

<400>  1
atgctccaag gccacagctc tgtgttccag gccttgctgg ggaccttctt cacctggggg      60

atgacagcag ctggggcagc tctcgtgttc gtattctcta gtggacagag gcggatctta     120

gatggaagtc ttggctttgc tgcaggggtc atgttggcag cttcctattg gtctcttctg     180

gccccagcag ttgagatggc cacgtcctct gggggcttcg gtgcctttgc cttcttccct     240

gtggctgttg gcttcaccct tggagcggct tttgtctact tggctgacct cctgatgcct     300

cacttgggtg cagcagaaga cccccagacg accctggcac tgaacttcgg ctctacgttg     360

atgaagaaga agtctgatcc tgagggtccc gcgctgctct tccctgagag tgaactttcc     420

atccggatag gtagagctgg gcttctttca gacaagagtg agaatggtga ggcatatcag     480

agaaagaagg cggcagccac tggccttcca gagggtcctg ctgtccctgt gccttctcga     540

gggaatctgg cacagcccgg cggcagcagc tggaggagga tcgcactgct catcttggcc     600

atcactatac acaacgttcc agagggtctc gctgttggag ttggatttgg ggctatagaa     660

aagacggcat ctgctacctt tgagagtgcc aggaatttgg ccattggaat cgggatccag     720

aatttccccg agggcctggc tgtcagcctt cccttgcgag ggcaggcttt ctccacctgg     780

agagctttct ggtatgggca gctgagcggc atggtggagc ccctggccgg ggtctttggt     840

gcctttgccg tggtgctggc tgagcccatc ctgccctacg ctctggcctt tgctgccggt     900

gccatggtct acgtggtcat ggacgacatc atccccgaag cccagatcag tggtaatggg     960

aaactggcat cctgggcctc catcctggga tttgtagtga tgatgtcact ggacgttggc    1020

ctgggc                                                               1026

<210>  2
<211>  20
<212>  DNA
<213>  Artificial Sequence

<220>
<223>  H11 sgRNA

<400>  2
ctgagccaac agtggtagta                                                   20


<210>  3
<211>  3
<212>  DNA
<213>  Artificial Sequence

<220>
<223>  PAM

<400>  3
agg                                                             3


<210>  4
<211>  20
<212>  RNA
<213>  Artificial Sequence

<220>
<223>  Zebrafish sgRNA sequence

<400>  4
ggagaagaug aagaccagug                                                   20


<210>  5
<211>  22
<212>  DNA
<213>  Artificial Sequence

<220>
<223>  CKO mouse ES cells 5′ homology arm forward primer

<400>  5
tgttgtttgc tcggaggctg tc                                                22


<210>  6
<211>  22
<212>  DNA
<213>  Artificial Sequence

<220>
<223>  CKO mouse ES cells 5′ homology arm reverse primer

<400>  6
ggcctacccg cttccattgc tc                                                22


<210>  7
<211>  22
<212>  DNA
<213>  Artificial Sequence

<220>
<223>  CKO mouse 5′ homology arm F2 generation de-Neo mouse identification
method

<400>  7
tgttgtttgc tcggaggctg tc                                                22


<210>  8
<211>  20
<212>  DNA
<213>  Artificial Sequence

<220>
<223>  CKO mouse 3′ homology arm F2 generation de-Neo mouse identification
method

<400>  8
gcccacccac cctccctact                                                   20


<210>  9
<211>  21
<212>  DNA
<213>  Artificial Sequence

<220>
<223> CKO mouse ES cells 3′ homology arm forward primer

<400>  9
ccgtgccttc cttgaccctg g                                                 21


<210>  10
<211>  23
<212>  DNA
<213>  Artificial Sequence

<220>
<223>  CKO mouse ES cells 3′ homology arm reverse primer

<400>  10
gcactctcca acaccagcct acc                                               23


<210>  11
<211>  24
<212>  DNA
<213>  Artificial Sequence

<220>
<223>  CKO mouse 3′ F2 generation de-Neo mouse identification method

<400>  11
catcagctgg ctgagtaggt tggt                                              24


<210>  12
<211>  23
<212>  DNA
<213>  Artificial Sequence

<220>
<223>  CKO mouse 3′ F2 generation de-Neo mouse identification method

<400>  12

gcactctcca acaccagcct acc                                              23

<210>  13
<211>  34
<212>  DNA
<213>  Artificial Sequence

<220>
<223>  Flox region

<400>  13
ataacttcgt ataatgtatg ctatacgaag ttat                                  34

<210>  14
<211>  1703
<212>  DNA
<213>  Artificial Sequence

<220>
<223>  PGK-Neo-polyA

<400>  14
tctaccgggt aggggaggcg cttttcccaa ggcagtctgg agcatgcgct ttagcagccc      60

cgctgggcac ttggcgctac acaagtggcc tctggcctcg cacacattcc acatccaccg     120

gtaggcgcca accggctccg ttctttggtg gccccttcgc gccaccttct actcctcccc     180

tagtcaggaa gttccccccc gccccgcagc tcgcgtcgtg caggacgtga caaatggaag     240

tagcacgtct cactagtctc gtgcagatgg acagcaccgc tgagcaatgg aagcgggtag     300

gcctttgggg cagcggccaa tagcagcttt gctccttcgc tttctgggct cagaggctgg     360

gaaggggtgg gtccgggggc gggctcaggg gcgggctcag gggcggggcg ggcgcccgaa     420

ggtcctccgg aggcccggca ttctgcacgc ttcaaaagcg cacgtctgcc gcgctgttct     480

cctcttcctc atctccgggc ctttcgacct gcagcctgtt gacaattaat catcggcata     540

gtatatcggc atagtataat acgacaaggt gaggaactaa accatgggat cggccattga     600

acaagatgga ttgcacgcag gttctccggc cgcttgggtg gagaggctat tcggctatga     660

ctgggcacaa cagacaatcg gctgctctga tgccgccgtg ttccggctgt cagcgcaggg     720

gcgcccggtt ctttttgtca agaccgacct gtccggtgcc ctgaatgaac tgcaggacga     780

ggcagcgcgg ctatcgtggc tggccacgac gggcgttcct tgcgcagctg tgctcgacgt     840

tgtcactgaa gcgggaaggg actggctgct attgggcgaa gtgccggggc aggatctcct     900

gtcatctcac cttgctcctg ccgagaaagt atccatcatg gctgatgcaa tgcggcggct     960

gcatacgctt gatccggcta cctgcccatt cgaccaccaa gcgaaacatc gcatcgagcg    1020

agcacgtact cggatggaag ccggtcttgt cgatcaggat gatctggacg aagagcatca    1080

ggggctcgcg ccagccgaac tgttcgccag gctcaaggcg cgcatgcccg acggcgatga    1140

tctcgtcgtg acccatggcg atgcctgctt gccgaatatc atggtggaaa atggccgctt    1200

ttctggattc atcgactgtg gccggctggg tgtggcggac cgctatcagg acatagcgtt 1260

ggctacccgt gatattgctg aagagcttgg cggcgaatgg gctgaccgct tcctcgtgct 1320

ttacggtatc gccgctcccg attcgcagcg catcgccttc tatcgccttc ttgacgagtt 1380

cttctgaggg gatcaattct ctagagctcg ctgatcagcc tcgactgtgc cttctagttg 1440

ccagccatct gttgtttgcc cctcccccgt gccttccttg accctggaag gtgccactcc 1500

cactgtcctt tcctaataaa atgaggaaat tgcatcgcat tgtctgagta ggtgtcattc 1560

tattctgggg ggtggggtgg ggcaggacag caagggggag gattgggaag acaatagcag 1620

gcatgctggg gatgcggtgg gctctatggc ttctgaggcg gaaagaacca gctggggctc 1680

gactagagct tgcggaaccc ttc 1703

<210> 15
<211> 1586
<212> DNA
<213> Artificial Sequence

<220>
<223> MC1-TK-polyA

<400> 15
gtcgagcagt gtggttttca agaggaagca aaaagcctct ccacccaggc ctggaatgtt 60

tccacccaat gtcgagcagt gtggttttgc aagaggaagc aaaaagcctc tccacccagg 120

cctggaatgt ttccacccaa tgtcgagcaa accccgccca gcgtcttgtc attggcgaat 180

tcgaacacgc agatgcagtc ggggcggcgc ggtcccaggt ccacttcgca tattaaggtg 240

acgcgtgtgg cctcgaacac cgagcgaccc tgcagcgacc cgcttaacag cgtcaacagc 300

gtgccgcaga tcttggtggc gtgaaactcc cgcacctctt cggccagcgc cttgtagaag 360

cgcgtatggc ttcgtacccc ggccatcagc acgcgtctgc gttcgaccag gctgcgcgtt 420

ctcgcggcca tagcaaccga cgtacggcgt tgcgccctcg ccggcagcaa gaagccacgg 480

aagtccgccc ggagcagaaa atgcccacgc tactgcgggt ttatatagac ggtccccacg 540

ggatggggaa aaccaccacc acgcaactgc tggtggccct gggttcgcgc gacgatatcg 600

tctacgtacc cgagccgatg acttactggc gggtgctggg ggcttccgag acaatcgcga 660

acatctacac cacacaacac cgccttgacc agggtgagat atcggccggg gacgcggcgg 720

tggtaatgac aagcgcccag ataacaatgg gcatgcctta tgccgtgacc gacgccgttc 780

tggctcctca tatcgggggg gaggctggga gctcacatgc cccgcccccg gccctcaccc 840

tcatcttcga ccgccatccc atcgccgccc tcctgtgcta cccggccgcg cgatacctta 900

tgggcagcat gaccccccag gccgtgctgg cgttcgtggc cctcatcccg ccgaccttgc 960

ccggcacaaa catcgtgttg ggggcccttc cggaggacag acacatcgac cgcctggcca 1020

aacgccagcg ccccggcgag cggcttgacc tggctatgct ggccgcgatt cgccgcgttt 1080

acgggctgct tgccaatacg gtgcggtatc tgcagggcgg cgggtcgtgg cgggaggatt 1140

ggggacagct ttcggggacg gccgtgccgc cccagggtgc cgagccccag agcaacgcgg 1200

gcccacgacc ccatatcggg gacacgttat ttaccctgtt tcgggccccc gagttgctgg 1260

cccccaacgg cgacctgtac aacgtgtttg cctgggcctt ggacgtcttg gccaaacgcc 1320

tccgtcccat gcacgtcttt atcctggatt acgaccaatc gcccgccggc tgccgggacg 1380

ccctgctgca acttacctcc gggatgatcc agacccacgt caccacccca ggctccatac 1440

cgacgatctg cgacctggcg cgcacgtttg cccgggagat gggggaggct aactgaaaca 1500

cggaaggaga caataccgga aggaacccgc gctatgacgg caataaaaag acagaataaa 1560

acgcacgggt gttgggtcgt ttgttc 1586